(19) Europäisches Patentamt
European Patent Office
Office européen des brevets

(11) **EP 3 112 017 A1**

(12) **EUROPEAN PATENT APPLICATION**
published in accordance with Art. 153(4) EPC

(43) Date of publication:
04.01.2017 Bulletin 2017/01

(51) Int Cl.:
*B01J 19/00* (2006.01)   *C09B 48/00* (2006.01)
*C09B 67/14* (2006.01)   *C09B 67/20* (2006.01)
*A61K 31/405* (2006.01)

(21) Application number: 15752000.8

(22) Date of filing: 18.02.2015

(86) International application number:
PCT/JP2015/054427

(87) International publication number:
WO 2015/125819 (27.08.2015 Gazette 2015/34)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 18.02.2014   JP 2014028354

(71) Applicant: M Technique Co., Ltd.
Osaka 594-1144 (JP)

(72) Inventor: ENOMURA Masakazu
Izumi-shi
Osaka 594-1144 (JP)

(74) Representative: Maury, Richard Philip
Marks & Clerk LLP
90 Long Acre
London WC2E 9RA (GB)

(54) **FINE PARTICLE PRODUCTION METHOD**

(57) This fine particle production method involves a dissolving step in which a stirrer having a rotating stirring blade is used to dissolve at least one type of fine particle raw material in a solvent to obtain a fine particle raw material solution, and a precipitation step in which the fine particle raw material solution and at least one type of precipitation solvent for precipitating the fine particle raw material from the fine particle raw material solution are introduced between at least two treatment surfaces which are arranged oppositely one another, can move closer to and farther apart from one another, and at least one of which can rotate relative to the other, and the fine particle raw material solution and the at least one type of precipitation solvent are mixed in a thin film fluid formed between the at least two treatment surfaces, and the fine particles are precipitated. The stirring energy is determined by the stirring time conditions of the stirrer, the circumferential velocity conditions of the stirring blade, and the temperature conditions of the fine particle raw material solution, and in the dissolving step, the stirring energy is varied by changing at least one of the aforementioned conditions, and by changing the stirring energy, the degree of crystallization and the crystal form of the fine particles obtained in the precipitation step are controlled.

Fig. 6

EP 3 112 017 A1

## Description

Technical Field

The present invention relates to a method for producing microparticles.

Microparticles of a metal, an oxide, a medicine, a food stuff, a biological ingesting material such as a cosmetic, a pigment, and so on are wanted in a wide range of the industrial field.

In a general method for producing microparticles, a poor solvent method, crystallization, or a reaction such as oxidation and reduction is carried out by using a flask, a beaker, a tank, and the like as described in Patent Document 1. However, when vessels like these are used, it is difficult to keep concentration and temperature uniformly in the vessels, so that the particle diameter distribution of the obtained microparticles tends to be broad; and in the case of producing microparticles containing two or more elements, such as a metal alloy and a composite oxide, it has been difficult to produce microparticles having a uniform element ratio. Alternatively, a method for producing microparticles which uses a microreactor as described in Patent Document 2 is provided; however, under the current situation, a general microreactor still has many problems to be solved, such as clogging by a reacted material and difficulty in up-scaling. In view of the above situation, a method for producing homogeneous and uniform microparticles stably and with a low energy and a low cost has been eagerly wanted.

By Applicant of the present invention, a method for producing microparticles, such as those described in Patent Document 3, was provided in which a microparticle raw material solution having a microparticle raw material dissolved therein is mixed with a separating solution to separate the microparticles in a thin film fluid formed between at least two processing surfaces which are disposed in a position they are faced with each other so as to be able to approach to and separate from each other, at least one of which rotates relative to the other.

However, even in the case that a method described in Patent Document 3 is used, there has been a certain instance that microparticles cannot be produced stably; and in the case that microparticles containing two or more molecules or elements are produced, there has been a certain instance that to produce homogeneous and uniform microparticles is difficult because of a local variance in the ratio of these elements.

Furthermore, even in the case that a method as described in Patent Document 4 is used together, it is difficult to achieve the desired degree of crystallinity, crystal form, and the component ratio of the particular crystal form for the produced microparticle only by simply changing the operation conditions of the apparatus such as the formulation, the liquid feeding amount, and the temperature of the fluids to be processed, and the number of rotations of the processing surface. Specific methods to freely control the characteristics and properties of the microparticles such as the degree of crystallinity, the crystal form, and the component ratio of the particular crystal form has not been disclosed, and there is room for improvement. Here, the "component ratio of the particular crystal form" is the ratio of the crystal component of the particular crystal form in the crystal component of a plurality of crystal forms when mircoparticles to be produced have a plurality of crystal forms.

PRIOR ART DOCUMENTS

PATENT DOCUMENT

Patent Document 1: Japanese Laid-Open Publication No. 2002-97281
Patent Document 2: Japanese Laid-Open Publication No. 2006-193652
Patent Document 3: International Patent Laid-Open Publication No. 2009/008393
Patent Document 4: International Patent Laid-Open Publication No. 2012/128273

Disclosure of Invention

Problems to be Solved by the Invention

The present invention was made in view of the circumstances as described above, and aims to provide a production method capable of obtaining desired microparticles having characteristics/properties such as the degree of crystallinity, the crystal form, and the component ratio of a particular crystal form.

The method for producing microparticles according to the present invention includes: a dissolving step of dissolving at least one type of microparticle raw material in a solvent by using an agitator having a rotating agitating blade to obtain a microparticle raw material solution; and a separating step of separating microparticles by introducing at least one type of separating solution to separate the microparticle raw material from the microparticle raw material solution and the microparticle raw material solution in between at least two processing surfaces, which are disposed in a position facing each other, so as to be able to approach to and separate from each other, at least one of which rotates relative to the other, and mixing them in a thin film fluid formed between the at least two processing surfaces.

Then, in the dissolving step, an agitation energy determined by an agitation time condition of the agitator, a peripheral velocity condition of the agitating blade, and a temperature condition of the microparticle raw material solution is increased or decreased by changing at least one of the conditions, so that a degree of crystallinity of the microparticles obtained in the separating step is controlled by the increase and decrease of the agitation energy.

Further, in the dissolving step, the agitation energy determined by the agitation time condition of the agitator, the peripheral velocity condition of the agitating blade, and the temperature condition of the microparticle raw material solution is increased or decreased by changing at least one of the conditions to control a crystal form of the microparticle obtained in the separating step by an increase or decrease of the agitation energy.

Alternatively, it may be carried out such that, in the dissolving step, ther agitation energy determined by the agitation time condition of the agitator, the peripheral velocity condition of the agitating blade, and the temperature condition of the microparticle raw material solution is increased or decreased by changing at least one of the conditions to control both of the degree of crystallinity and the crystal form of the microparticle obtained in the separating step by an increase or a decrease of the agitation energy.

Here, the agitation energy will be explained in more detail.

First of all, a power P of the agitator (amount of work per unit of time) can be calculated by the following formula (1).

$$\text{Agitation Power } P[kw] = Np \times \rho \times n^3 \times d^5 \quad \text{Formula (1)}$$

Np: Power Factor (dimensionless number calculated from experimental data. For example, Np=0.95 to 1.05 in the case of CLEARMIX (produced by M Technique Co., Ltd.)

$\rho$: density [kg/m$^3$]

n: number of rotations [rps]

d: rotor diameter [m]

Next, the agitation energy (that is, the energy applied for agitation) can be expressed by the product of the agitation power and the agitation time (agitation power P[kw] $\times$ agitation time t[s]) as follows.

$$\text{Agitation energy} = Np \times \rho \times n^3 \times d^5 \times t \quad \text{Formula (2)}$$

Furthermore, because of the relationship of the peripheral velocity v = $\pi \times$d$\times$n, the above-mentioned formula (2) can be rewritten as follows.

$$\text{Agitation energy} = Np \times (1/\pi^3) \times \rho \times v^3 \times d^2 \times t \quad \text{Formula (3)}$$

Here, the processing amount of the raw material solution and the container size containing the raw material solution are unified, and when using the same agitator, it can be considered as an identical system and since the rotor diameter d[m] is constant, $Np \times (1/\pi^3) \times d^2$ can be used as a constant number.

In a common solution, when the temperature increases, the solution expands and its volume increases, and therefore the density decreases. On the other hand, when the temperature decreases, the solution shrinks and its volume decreases, and therefore the density increases. Thus, the density of the raw material solution changes according to the temperature. That is, the density of the raw material solution depends on the temperature.

Therefore, according to Formula (3), the agitation energy is determined by the agitation time condition, the peripheral velocity condition of the agitating blade, and the temperature condition of the microparticle raw material solution.

As the separating method of the microparticles in the separating step, although not specifically limited, an acid pasting method, an alkaline pasting method, and a poor solvent method can be exemplified as representatives. Prior to the separating step by a separating method represented by the exemplified separating method, by increasing the agitation energy in the dissolving step, it may be carried out to control so that the ratio of the degree of crystallinity of the microparticle to a particle diameter of the microparticle increases by increasing the agitation energy in the dissolving step.

Furthermore, the present invention is configured to separate the microparticles in the separating step by various separating methods represented by the exemplified separating method, and the microparticle has a plurality of crystal forms, a ratio of a crystalline component of a particular crystal form to a crystalline component of a plurality of crystal forms is a component ratio of the particular crystal form. The present invention can be carried out to control so that the ratio of the component ratio of the particular crystal form to the particle diameter of the microparticle increases by increasing the agitation energy in the dissolving step.

Further, the present invention can be carried out in a manner in which the microparticles are pigment microparticles.

Pigment particles are used in a very wide variety of fields, such as fields of coating, printer ink, toner, inkjet ink, color filter, and the like, but as one of fields in which a practically high-function material is needed, there are a field of a color filter for an image sensor and a color filter for a liquid crystal display (hereinafter referred to as "LCD"). High transmittance characteristics are required for pigments for LCD color filters, and as indexes regarding the transmittance characteristics, there are a "degree of crystallinity" and a "component ratio of a particular crystal form". Pigment microparticles high in transmittance can be obtained by controlling these indexes.

Further, in the case of an inkjet ink and a pigment for a toner, color hue, coloring power, and durability are required, and as the indexes to evaluate them, there are a "degree of crystallinity" and a "component ratio of a particular crystal form".

As one example, there exit $\alpha$-type crystals and ß-type crystals for the crystal type of 2,9-dimethylquinacridone (C.I.Pigment Red 122) (hereinafter referred to as "PR122") , normally both exist, and the ß-type crystal is a stable form and the $\alpha$-type crystal is a metastable form. The higher the ratio of the $\alpha$-type crystal is, it is more yellowish. It is required that the ratio of the crystal form is determined according to the intended color tone. The ratio of the $\alpha$-type crystal or the ß-type crystal occupied in the crystal component in PR122 in which the $\alpha$-type crystal and the ß-type crystal are mixed as mentioned above is referred to as a "component ratio of a particular crystal form". Among them, the ratio of the ß-type crystal occupied in the crystal component in the PR122 in which the $\alpha$-type crystal and the ß-type crystal are mixed is referred to as a "ß-type crystal component ratio".

Further, in PR122 as a whole, both crystal components and amorphous coexist, and the occupied ratio of the crystallized component to the entirety of the crystallized component and the amorphous is referred to as a "degree of crystallinity". It is well known that normally, the higher the degree of crystallinity is, the durability against light, heat, humidity, and the like improves.

That is, by controlling the degree of crystallinity of the microparticles obtained in the separating step by the increase and decrease of the agitation energy in the dissolving step, and by controlling the component ratio of a particular crystal form of microparticles obtained in the separating step by the increase and the decrease of the agitation energy in the dissolving step, the PR122 microparticles used as an inkjet ink need to be individually created to have a high "degree of crystallinity" and the "$\alpha$-type crystal ratio" and the "ß-type crystal ratio" have to be individually created according to the intended color tone.

Further, the present invention can be carried out in a manner in which the microparticles are something other than pigment microparticles. In the case of indomethacin which is a pharmaceutical product, by controlling the degree of crystallinity of the microparticles obtained in the separating step by the increase and decrease of the agitation energy in the dissolving step, and by controlling the component ratio of a particular crystal form of the microparticles obtained in the separating step by the increase and decrease of the agitation energy in the dissolving step, a desired indomethacin microparticles can be obtained with regards to the characteristics/properties such as a degree of crystallinity and a crystal form.

Indomethacin has a plurality of crystal forms, and is typically represented by a stable form $\gamma$-type crystal, a metastable form $\alpha$-type crystal, and a metastable form ß-type crystal. Typically, these crystals forms coexist, but the higher the ratio of the $\gamma$-type crystal is, the more stable the properties become. The occupied ratio of the $\gamma$-type crystal, $\alpha$-type crystal, and ß-type crystal in the crystal component in the indomethacin in which the $\gamma$-type crystal, the $\alpha$-type crystal, and the ß-type crystal are mixed is referred to as a "component ratio of a particular crystal form". Among them, the ratio of the $\gamma$-type crystal occupied in the crystal component in the indomethacin in which the $\gamma$-type crystal, the $\alpha$-type crystal, and the ß-type crystal are mixed is referred to as a "$\gamma$-type crystal component ratio."

As with PR122 which is a pigment and indomethacin which is a pharmaceutical product, even in the case of compounds different in application, the degree of crystallinity of microparticles obtained in the separating step can be controlled by the increase and decrease of the agitation energy in the dissolving step, and the crystal form of microparticles obtained in the separating step can be controlled by the increase and decrease of the agitation energy in the dissolving step. Therefore, even in other substances, it is considered that it indicates similar tendencies.

In the present invention, the so-called melted state can be controlled by increasing and decreasing the agitation energy, which is a physical energy, in the dissolving step. In other words, the melted state of microparticle raw material solution can be changed, the cluster shape state can be changed, and a microparticle raw material solution in a melted state or in a molecular dispersion state at a molecular level can be prepared. Therefore, the degree of crystallinity and the crystal forms of microparticles (the component ratio of a particular crystal form when a plurality of crystal forms exist) obtained in the separating step, which is a later step, can be controlled. Therefore, in preparing a microparticle raw material solution, since it is not particularly affected by the change of the type of a substance constituting the compound and the type of chemical reaction, it is considered that it indicates similar tendencies even in the case of substances other than PR122 and indomethacin.

Further, in the present invention, in producing microparticles in which the particle diameter, the degree of crystallinity, and the crystal form are set to particular conditions, by changing one of the conditions among the peripheral velocity condition, the agitation time condition, and the temperature condition in the dissolving step (first condition) and fixing the other two conditions (second and third conditions), the first condition satisfying the particular conditions for at least one of the particle diameter, the degree of crystallinity, and the crystal form of the microparticles in the separating step is determined. The present invention can be carried out by changing at least either one of the second and third conditions while maintaining the determined first condition to determine at least either one of the second and third conditions for the remaining two that are different from the above-mentioned at least one of the particle diameter, the degree of crystallinity, and the crystal form of the microparticles in the separating step, thereby producing microparticles in which the particle diameter, the degree of crystallinity, and the crystal form satisfy the particular conditions.

As an example, the present invention can be carried out in a manner in which, in producing microparticles in which the particle diameter, the degree of crystallinity, and the crystal form are set to particular conditions, by changing the peripheral velocity condition of the agitating blade in the dissolving step, the peripheral velocity condition satisfying the particular condition for the particle diameter of the microparticles in the separating step is determined, and by changing at least either one of the agitation time condition and the temperature condition while maintaining the determined peripheral velocity condition to determine the agitation time condition and the temperature condition satisfying the particular conditions for the degree of crystallinity and the crystal form of the microparticles in the separating step, microparticles in which the particle diameter, the degree of crystallinity, and the crystal form satisfy the particular conditions are produced.

According to a mere one embodiment of the present invention shown above, this method can be executed as the method for producing microparticles, wherein

at least two types of fluids to be processed are used,

of them, at least one type of fluid to be processed is the microparticle raw material solution, and

at least one type of fluid to be processed other than the microparticle raw material solution is the separating solvent, wherein provided therewith are:

a fluid pressure imparting mechanism for imparting a pressure to the fluids to be processed,
a first processing member provided with a first processing surface of at least two processing surfaces,
a second processing member provided with a second processing surface of the at least two processing surfaces, and
a rotation drive mechanism for rotating these processing members relative to each other, wherein
each of the processing surfaces constitutes part of a closed flow path through which the fluids to be processed under the pressure is passed,
of the first processing member and the second processing member, at least the second processing member is provided with a pressure-receiving surface, and at least part of the pressure-receiving surface is comprised of the second processing surface, wherein
this pressure-receiving surface receives a pressure applied to the fluids to be processed by the fluid pressure imparting mechanism, thereby generating a force to move in the direction of separating the second processing surface from the first processing surface, and
the fluids to be processed under the pressure are introduced into between the first processing surface and the second processing surface which are disposed in a position they are faced with each other so as to be able to approach to and separate from each other, at least one of which rotates relative to the other, thereby forming the thin film fluid by the fluids to be processed,
thereby separating the microparticles in this thin film fluid.

According to a mere one embodiment of the foregoing present invention, this method can be executed as the method for producing microparticles, wherein

of the fluids to be processed, at least any one type of the fluids passes through between both the processing surfaces while forming the thin film fluid,

at least one separate introduction path independent of a flow path through which the any one type of the fluids to be processed passes is arranged,

at least one opening which leads to the introduction path is provided to at least either one of the first processing surface and the second processing surface,

at least one type of fluid which is different from the at least any other one type of the fluids is introduced between the processing surfaces through the opening, whereby mixing these fluids to be processed in the thin film fluid,

thereby separating the microparticles in this thin film fluid.

Effects of the Invention

According to the present invention, it is possible to control a degree of crystallinity and a crystal form of microparticles, and microparticles in which the degree of crystallinity and the crystal form are controlled can be continuously produced.

Further, according to the present invention, the preparation of the microparticles raw material solution is performed using an agitator having rotating agitating blades. In doing so, with a simple change of a processing condition in which the agitation energy is increased or decreased, at least one of the three conditions defining the agitation energy (agitation time, peripheral velocity of the agitating blade, the temperature of the microparticles raw material solution) is changed to control the so-called melted state, that is, to change the melted state of the microparticle raw material solution, to change the cluster shape state, and to prepare a microparticle raw material solution in a melted state or in a molecular dispersion state at a molecular level. Therefore, the degree of crystallinity and the crystal form of the microparticles separated in the later separating step can be controlled, which in turn can obtain desired microparticles with respect to chracteristics/properties such as the degree of crystallinity and crystal form.

Furthermore, microparticles according to the purpose can be selectively produced.

[FIG. 1] FIG. 1 is a schematic sectional view showing a fluid processing apparatus according to an embodiment of the present invention.

[FIG. 2] FIG. 2 (A) is a schematic plane view of a first processing surface in the fluid processing apparatus shown in FIG. 1, and FIG. 2(B) is an enlarged view showing an important part of the processing surface in the apparatus.

[FIG. 3] FIG. 3(A) is a sectional view of a second introduction part of the apparatus, and FIG. 3(B) is an enlarged view showing an important part of the processing surface for explaining the second introduction part.

[FIG. 4] FIG. 4 is a front view of an agitator having agitating blades that rotate according to the embodiment of the present invention.

[FIG. 5] This shows an explanatory drawing of an internal structure of the agitator.

[FIG. 6] FIG. 6 is a graph showing changes of the degree of crystallinity / average particle diameter and the ß-type crystal ratio/average particle diameter of PR122 microparticles with respect to the preparation temperature of the second fluid in Examples 1 to 3.

[FIG. 7] FIG. 7 is a graph showing changes of the degree of crystallinity/average particle diameter and the ß-type crystal ratio/average particle diameter of PR122 microparticles with respect to the preparation time of the second fluid in Examples 4 to 7.

[FIG. 8] FIG. 8 is a graph showing changes of the degree of crystallinity/average particle diameter and the ß-type crystal ratio/average particle diameter of PR122 microparticles with respect to the preparation time of the second fluid in Examples 8 to 11.

[FIG. 9] FIG. 9 is a graph showing changes of the degree of crystallinity / average particle diameter and the ß-type crystal ratio/average particle diameter of PR122 microparticles with respect to the preparation time of the second fluid in Examples 12 to 16.

[FIG. 10] FIG. 10 is a graph showing changes of the degree of crystallinity/average particle diameter of PR122 microparticles with respect to the preparation time of the second fluid in Examples 8 to 11, 12, 13, 15, and 16.

[FIG. 11] FIG. 11 is a graph showing changes of the ß-type crystal ratio/average particle diameter of PR122 microparticles with respect to the preparation time of the second fluid in Examples 8 to 11, 12, 13, 15, and 16.

[FIG. 12] FIG. 12 is a graph showing changes of the degree of crystallinity/average particle diameter of PR122 microparticles with respect to the peripheral velocity at the time of preparing the second fluid in Examples 17 to 22.

[FIG. 13] FIG. 13 is a graph showing changes of the ß-type crystal ratio/average particle diameter of PR122 microparticles with respect to the peripheral velocity at the time of preparing the second fluid in Examples 17 to 22.

[FIG. 14] FIG. 14 is a graph showing changes of the degree of crystallinity/average particle diameter of PR122 microparticles with respect to the preparation time of the second fluid in Examples 23 to 31.

[FIG. 15] FIG. 15 is a graph showing changes of the ß-type crystal ratio/average particle diameter of PR122 microparticles with respect to the preparation time of the second fluid in Examples 23 to 31.

[FIG. 16] FIG. 16 is a graph showing changes of the degree of crystallinity/average particle diameter of PR122 microparticles with respect to the preparation temperature of the second fluid in Examples 23 to 31.

[FIG. 17] FIG. 17 is a graph showing changes of the ß-type crystal ratio/average particle diameter of PR122 microparticles with respect to the preparation temperature of the second fluid in Examples 23 to 31.

[FIG. 18] FIG. 18 is a graph showing changes of the degree of crystallinity/average particle diameter of PR122 microparticles with respect to the preparation time of the second fluid in Examples 32 to 40.

[FIG. 19] FIG. 19 is a graph showing changes of the ß-type crystal ratio/average particle diameter of PR122 microparticles with respect to the preparation time of the second fluid in Examples 32 to 40.

[FIG. 20] FIG. 20 is a graph showing changes of the degree of crystallinity/average particle diameter of PR122 microparticles with respect to the preparation temperature of the second fluid in Examples 32 to 40.

[FIG. 21] FIG. 21 is a graph showing the ß-type crystal ratio/average particle diameter of PR122 microparticles with respect to the preparation temperature of the second fluid in Examples 32 to 40.

[FIG. 22] FIG. 22 is a graph showing changes of the degree of crystallinity/average particle diameter of indomethacin microparticles with respect to the preparation time of the second fluid in Examples 41 to 49.

[FIG. 23] FIG. 23 is a graph showing changes of the degree of crystallinity/average particle diameter of indomethacin microparticles with respect to the preparation temperature of the second fluid in Examples 41 to 49.

[FIG. 24] FIG. 24 is a graph showing changes of the $\gamma$-type crystal ratio/average particle diameter of indomethacin microparticles with respect to the preparation time of the second fluid in Examples 41 to 49.

[FIG. 25] FIG. 25 is a graph showing changes of the $\gamma$-type crystal ratio/average particle diameter of indomethacin microparticles with respect to the preparation temperature of the second fluid in Examples 41 to 49.

Embodiments for Carrying Out the Invention

Hereinafter, detailed explanation of the present invention will be made; but a technical range of the present invention is not limited by the following Embodiments and Examples.

In the present invention, there is no particular restriction as to the kind of the microparticles. One example thereof includes an organic substance, an inorganic substance, and a composite substance of an organic substance and an inorganic substance. The other example includes a metal and/or a non-metal, and a compound of them. Although there is no particular restriction as to the compound of a metal and/or a non-metal, one example thereof includes a metal or a non-metal in the form of a salt, an oxide, a hydroxide, a hydroxylated oxide, a nitride, a carbide, a complex, an organic salt, an organic complex, an organic compound, and a hydrate and an organic solvent adduct of them. Although not particularly restricted, included are a nitrate salt, a nitrite salt, a sulfate salt, a sulfite salt, a formate salt, an acetate salt, a phosphate salt, a phosphite salt, a hypophosphite salt, a chloride, an oxy salt, an acetylacetonato salt of a metal or non-metal, and a hydrate and an organic solvent adduct of them.

In the present invention, a poor solvent method to separate, precipitate or crystalize the above-mentioned microparticles or a reaction such as an oxidation reaction and a reducing reaction is carried out in a thin film fluid formed between at least two processing surfaces which are disposed in a position they are faced with each other so as to be able to approach to and separate from each other, at least one of which rotates relative to the other, so that the microparticles can be produced. Specifically, a microparticle raw material solution having a microparticle raw material of intended microparticles mixed with or dissolved in a solvent and a separating solution to separate the microparticle raw material are introduced in between at least two processing surfaces which are disposed in a position they are faced with each other so as to be able to approach to and separate from each other, at least one of which rotates relative to the other, and mixed in the thin film fluid formed between the at least two processing surfaces, thereby separating the microparticles.

As to the microparticle raw material in the present invention, the same material as the microparticles mentioned above may be used. The microparticle raw material solution in the present invention is prepared by mixing or dissolving (hereinafter simply referred to as "dissolving") at least one type of microparticle raw material in a solvent. Preferably, the present invention is carried out by dissolving or dispersing at least one type microparticle raw material into a solvent.

When using a pigment as a microparticle raw material in the present invention, although not particularly limited, examples can be exemplified by an organic-pigment, an inorganic pigment, an organic-inorganic composite pigment, and all pigments registered in The Society of Dyers and Colorists, and the like.

The organic pigment in the present invention is not particularly limited, but examples include a perylene compound pigment, a perynone compound pigment, a quinacridone compound pigment, a quinacridone quinone compound pigment, an anthraquinone compound pigment, an anthanthrone compound pigment, a benzimidazolone compound pigment, a disazo condensation pigment compound, a disazo compound pigment, an azo compound pigment, an indanthrone compound pigment, a phthalocyanine compound pigment, a triaryl carbonium compound pigment, a dioxazine compound pigment, an aminoanthraquinone compound pigment, a thioindigo compound pigment, an isoindoline compound pigment, an isoindolinone compound pigment, a pyranthrone compound pigment, an isoviolanthrone compound pigment, and a compound of them.

As to the inorganic pigment in the present invention, although not particularly limited, one example includes metal compounds, and the like. Although not particularly limited, bengala, black iron oxide, yellow oxide-based compound, titanium oxide and zinc oxide as a white pigment, prussian blue, ultramarine, chromium oxide, magnesium oxide, aluminum oxide, calcium oxide, zirconium oxide, sulfide of cadmium, and zinc, and all metal compounds such as other inorganic pigments and inorganic compounds in general can be exemplified.

As a solvent to dissolve the microparticle raw material, for example, water, an organic solvent, or a mixed solvent comprising a plurality of them can be exemplified. As the water, tap water, ion-exchanged water, pure water, ultrapure water, RO water can be exemplified. As the organic solvent, an alcohol-based solvent, an amide-based solvent, a ketone-based solvent, an ether-based solvent, an aromatic-based solvent, a carbon disulfide, an aliphatic-based solvent, a nitrile-based solvent, a sulfoxide-based solvent, a halogen-based solvent, an ester-based solvent, an ionic liquid, a carboxylic acid compound, a sulfonic acid compound, and the like can be exemplified. The above-mentioned solvents may be used separately or as a mixture of a plurality of them.

Alternatively, the present invention can be carried out by mixing or dissolving a basic substance or an acidic substance in the abovementioned solvent. As the basic substance, a metal hydroxide such as a sodium hydroxide and a potassium hydroxide, a metal alkoxide such as a sodium methoxide and a sodium isopropoxide, and an amine-based compound such as trimethylamine, 2-diethylaminoethanol, and diethylamine. As he acidic substance, an inorganic acid such as aquaregia, hydrochloric acid, nitric acid, fuming nitric acid, sulfuric acid, and fuming sulfuric acid; and an organic acid such as formic acid, acetic acid, chloroacetic acid, dichloroacetic acid, oxalic acid, trifluoroacetic acid, and trichloroacetic acid can be exemplified. These basic substances or acidic substances may be used by mixing with the above-mentioned various solvents, or may be respectively used solely.

Alternatively, the present invention can be carried out by mixing or dissolving an oxidizing agent or a reducing agent in the abovementioned solvents. As the oxidizing agent, although not particularly limited, a nitrate salt, a hypochlorite salt, a permanganate salt, and a peroxide can be exemplified. As the reducing agent, an aluminum lithium hydride, sodium borohydride, hydrazine or a hydrate of hydrazine, a sulfite ion, and a metal ion, especially a transition metal ion (such as an iron ion and a titanium ion) can be exemplified.

As to the separating solvent to separate the microparticle raw material by mixing with the above-mentioned microparticle raw material solution, the same solvent as those mentioned before may be used. With regard to the solvent to dissolve the microparticle raw material and the solvent to separate it, the present invention may be carried out by appropriately selecting the solvent for dissolution and the solvent for separation in accordance with the intended microparticles.

In the present invention, it is preferable that the preparation of the microparticle raw material solution be performed by using an agitator having agitating blades that rotate. Specifically, in obtaining a microparticle raw material solution by dissolving at least one type of microparticle raw material in the solvent, an agitator having agitating blades that rotate is used. By doing so, not only the generation of coarse particles previously caused by undissolved substances in the microparticle raw material solution can be suppressed, but also, even in the case of dissolving two or more types of molecules or elements, a microparticle raw material solution in a more uniform dissolution state can be obtained promptly.

The inventor presumes that the uniform dissolution state cannot be obtained only by simply dissolving microparticle raw materials in the solution in the pre-treatment, but by preparing the microparticle raw material solution by using an agitator having agitating blades that rotate, a microparticle raw material solution in a uniform dissolution state at a molecular level or a molecular dispersion state can be obtained, which can improve the dissolution state or the cluster forming state of the microparticle raw material solution. In fact, the present inventor performed experiments repeatedly by trial and error by preparing a microparticle raw material solution by changing the conditions of the agitator, introducing the prepared microparticle raw material solution and the separating solvent in between at least two processing surfaces which are disposed in a position they are faced with each other so as to be able to approach to and separate from each other, at least one of which rotates relative to the other, and mixing them in the thin film fluid formed between at least two processing surfaces to separate the microparticles. As a result, it was a big surprise to see a relationship between the increase and decrease of the agitation energy due to the change in the conditions of the agitator, and the particle diameter, the degree of crystallinity, and the crystal form of the microparticles. Here, as the various conditions of the agitator, at least one among the agitation time by the agitator, the peripheral velocity of the agitating blade, and the temperature of the microparticle raw material solution was changed to increase and decrease the agitation energy.

Various embodiments of the present invention may be exemplified. As one example, in separating the microparticles by an acid pasting method, by increasing the agitation energy by changing the conditions of the agitator, it is possible to control such that the ratio of the degree of crystallinity to the particle diameter of the separated microparticles increases.

As another example, in separating the microparticles by the acid pasting method, by increasing the agitation energy by changing the conditions of the agitator, it is possible to control such that the component ratio of the particular crystal form to the particle diameter of the separated microparticles increases.

Further, as still another example, in separating the microparticles by an alkaline pasting method, by increasing the agitation energy by changing the conditions of the agitator, it is possible to control such that the ratio of the degree of crystallinity to the particle diameter of the separated microparticles increases.

Furthermore, as still yet another example, in separating the microparticles by the alkaline pasting method, by increasing the agitation energy by changing the conditions of the agitator, the component ratio of the particular crystal form to the particle diameter of the separated microparticles increases.

Agitation and mixing by using a general stirring bar for a long period of time is not desirable because there occurs a problem such as partial decomposition of a molecule or an ion contained in the microparticle raw material; but this does not restrict the time for agitation using an agitator that rotates in the present invention.

The agitator in the present invention is not specifically limited as long as it is an agitator having rotating agitating blades. In a typical agitator having agitating blades that rotate, it is said to be a high speed rotation when the peripheral velocity at the tip end of the agitating blade is 1 m/sec or more. A method for the agitation in the present invention is not particularly restricted; the present invention may be carried out by using an agitator, a dissolving machine, an emulsifying machine, a dispersing machine, homogenizer, and so on with various types such as a shearing type, a friction type, a high pressure jet type, and a ultrasonic wave type. Illustrative example thereof includes a continuous emulsifying machine such as Uraltra Turrax (manufactured by IKA WORKS, Inc.), TK Homomixer (manufactured by Primix Corp.), TK Homomicline Flow and Filmix (both manufactured by Primix Corp.); and a batch or a continuous dual emulsification machine such as Clearmix (manufactured by M. Technique Co., Ltd.), Clearmix Dissolver (manufactured by M. Technique Co., Ltd.). It is also possible to prepare the microparticle raw material solution by using an ultrasonic homogenizer, an ultrasonic cleaning machine, a high pressure homogenizer, and the like.

Next, as described above, various embodiments of the agitator having agitating blades that rotate may be used. As one example, an agitator can be exemplified in which an agitation room having a screen in which a plurality of discharge ports are formed and agitating blades that rotate in the agitation room are provided, and the agitating blade is configured to rotate with the tip end thereof kept a minute distance with respect to the inner surface of the screen. The screen and the agitating blade are not limited as long as both of them can rotate relatively. It may be configured such that the screen rotates in a direction opposite to the rotation direction of the agitating blade or that the screen is fixed so as not to rotate.

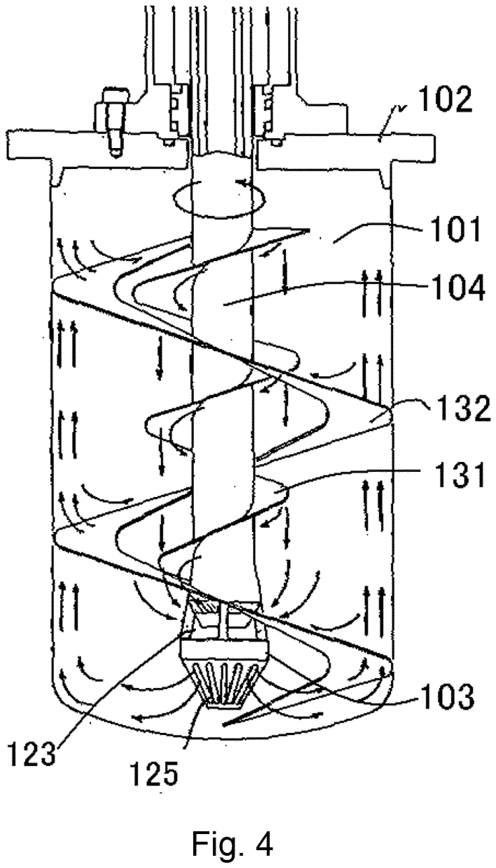

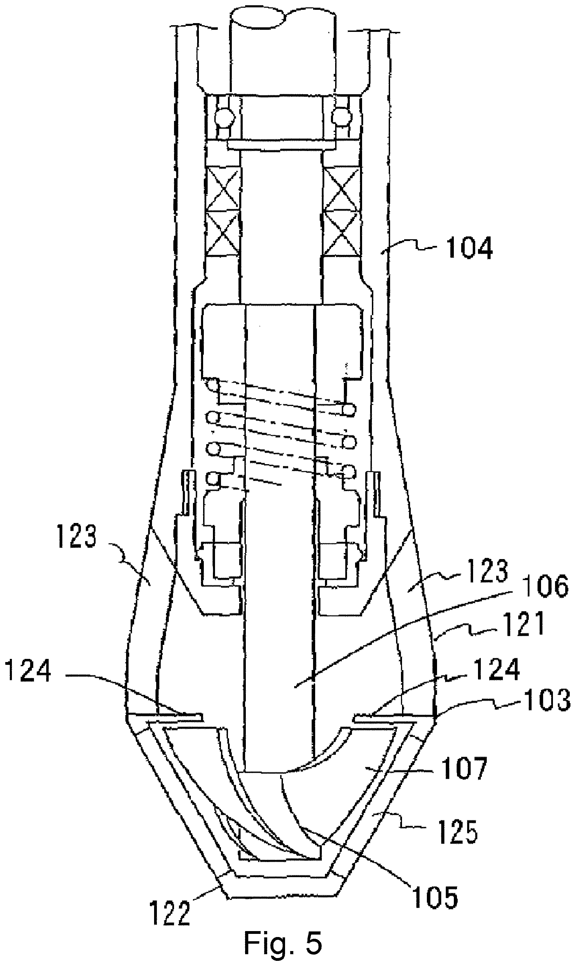

The agitator according to this embodiment will be described in more detail with reference to FIG. 4 and FIG. 5.

The agitator having agitating blades that rotate is, as shown in FIG. 4, inserted through the cover 102 into the holding tank 101 for holding a fluid to be processed. Hereinafter, in FIG. 4 and FIG. 5, the agitating blade will be referred to as a blade 107.

As shown in FIG. 5, the agitator having agitating blades that rotate is provided with the agitation room 103 and the supporting trunk 104 to support this agitation room 103. Inside the agitation room 103 is accommodated the impeller 105. This impeller 105 is arranged at the front end of the rotation shaft 106, and the rotation shaft 106 is arranged inside the supporting trunk 104. The rotation shaft 106 and the impeller 105 rotate in the reverse direction relative to the supporting trunk 104 and the agitation room 103. The respective base ends of the supporting trunk 104 and the rotation shaft 106 are connected to different rotation drive mechanisms (not shown by the figure).

The agitation room 103 is provided with the housing 121 which is arranged at the front end of the supporting trunk 104 and with the screen 122 which is arranged in the front end side of the housing 121. In the housing 121 is formed the admission port 123, and in the screen 122 is formed the discharge port 125. The fluid to be processed is introduced into the agitation room 103 thorough this admission port 123 by rotation of the impeller 105; and then, the fluid to be processed is discharged outside through the discharge port 125 after dispersion, dissolution, and so on. Alternatively, the discharge port 125 may be used as the admission port and the admission port 123 may be used as the discharge port. To compartmentalize between inside the screen 122 and inside the housing 121, the partition 124 may be arranged, though it may not be necessary.

Especially, the front end of the blade 107 of the impeller 105 is arranged along the inner wall of the screen 122 so as to maintain a minute space. The minute space is preferably set to around 0.2 to 2 mm. In the minute space, a large shearing force is applied to the fluid to be processed and kinetic energy is applied to the fluid to be processed by the rotation of the impeller 105, and the pressure of the fluid to be processed increases at the front of the rotation direction of the blade 107. And when the high-pressure fluid to be processed passes the discharge port 125, it is further accelerated and discharged outside the screen 122 while forming an intermittent jet stream. On the other hand, a negative pressure is generated at the rear of the rotation direction of the blade 107, and immediately after the blade 107 passes the discharge port 125, the fluid to be processed is absorbed inside the screen 122 from the discharge port 125. A large shearing force is generated between the fluids to be processed from the forward and reverse flows of the fluids to be processed.

The above-mentioned effect is achieved by relatively rotating the agitation room 103 having the screen 122 and the impeller 105. Specifically, it can be achieved by rotating the blade 107, which is an agitating blade, inside the agitation room 103 in a resting state. Further, like the above-mentioned example, the present invention can be carried out by rotating the agitation room 103 and the impeller 105 in the opposite direction relatively to rotate the discharge port 125 in a direction opposite to the rotation direction of the impeller 105. By doing so, the relative number of rotations between these two can be increased; and as a result, the capacity of the shearing treatment of the fluid to be processed may be enhanced.

The present invention is not limited to the above-mentioned embodiment; the screen 122 having the discharge port 125 may be removed, and only the housing 121 having the admission port 123 whereby rotating this housing may be arranged. By removing the screen 122, the fluid to be processed may be dissolved in a short period of time while controlling cavitation without applying the shearing force to the fluid to be processed. However, it is more preferable to provide the screen 122 at the front end side of the housing 121 because an intermittent jet stream occurs. Since the impeller 105 and the screen 122 rotates relatively, shearing of the fluid to be processed is performed in the minute space between the inner wall of the screen 122 including the discharge port 125 and the front edge of the blade 107, and the fluid to be processed is thereby discharged outside from the inside of the screen 122 as an intermittent jet stream via the discharge port 125. In the preparing the microparticle raw material solution, the present inventor presumes that the intermittent jet stream effectively acts for the dissolution of the microparticle raw material in the solvent, and the microparticle raw material solution is in a molecular level dissolution state or molecular dispersion state at the molecular level.

Because either one or both of the admission port 123 and the discharge port 125 arranged in the agitation room 103 rotate as mentioned above, any of admission and discharge or both of the fluid to be processed may sequentially change the positions thereof relative to the fluid to be processed present outside the agitation room 103; and as a result, alienation of the fluid to be processed from the circulation can be prevented from occurring. Alternatively, only the impeller 105 may be rotated nakedly without arranging the agitation room 103.

In order to securely circulate the fluid to be processed entirely in the holding tank 101, the introduction fin 131 which is rolled spirally along the longitudinal direction of the supporting trunk 104 may be arranged. By rotation of this introduction fin 131 together with the supporting trunk 104, the fluid to be processed present in the upper part of the holding tank 101 descends along the outer periphery of the supporting trunk 104 thereby introducing the fluid to be processed to the admission port 123. Alternatively, the circulation fin 132 which is rolled in the opposite direction to the introduction fin 131 may be arranged. This circulation fin 132 is disposed outside the introduction fin 131 so that the fluid to be processed that is discharged from the discharge port 125 is circulated to the upper part of the holding tank 101.

Further, the agitator shown in FIG. 4 and FIG. 5 is commercialized as the above-mentioned CLEARMIX (manufactured by M. Technique Co., Ltd.). Furthermore, a CLEARMIX DISSOLVER (manufactured by M. Technique Co. Ltd.) in which the screen is removed from the above-mentioned CLEARMIX (manufactured by M. Technique Co. Ltd.) may be used.

As described above, in a typical agitator, it is said to be a high speed rotation when a peripheral velocity at the front end of the agitating blade is 1 m/sec. or more. However, in the above-mentioned CLEARMIX or CLEARMIX DISSOLVER, it is preferable that a high speed rotation of a peripheral velocity of 31.42 m/sec or more at the front end of the agitating blade be performed to obtain an excellent agitation state.

The blade 107 of the impeller 105, in the cross-section (cross-section orthogonal to the axial direction of the rotation shaft 106), may extend radially and linearly at a constant width from the center of the impeller 10, gradually increased in width toward the outside, or extend outward in a curved manner. Further, in the axial direction of the rotation shaft 106, these blades 107 may extend linearly along the plane including the rotation axis of the rotation shaft 106, or extend in a warped manner in the up-and-down direction such as a spiral shape.

Furthermore, the maximum outer diameter of the blade 107 of the impeller 105 may be arbitrarily set according to embodiments.

Further, the discharge port 125 is shown to extend linearly in the axial direction of the rotation shaft 106 (up-and-down direction in the example illustrated in the drawing), but it may also extend in a curved manner such as in a spiral shape. Furthermore, the shape of the discharge port 125 does not necessarily have to be a narrow and thin space, and may be a polygonal, circular, or elliptical shape. Also, in the circumferential direction, although a plurality of discharge ports 125 are formed at even intervals, they may be formed by displacing the intervals, and the discharge ports 125 are not restricted from being provided in a variety of shapes and sizes.

Further, the present invention can be carried out by using an agitator having a typical agitating blade and is not restricted to the use of the above-mentioned CLEARMIX or CLEARMIX DISSOLVER. At that time, the peripheral velocity of the agitating blade in dissolving the microparticle raw material is not particularly restricted, but the velocity of 1 m/sec. or more is preferable. The velocity may be appropriately chosen in accordance with the viscosity and the temperature of the solvent, or the concentration of the microparticle raw material to be dissolved.

In the present invention, the peripheral velocity of the agitating blade refers to the moving speed of the maximum outer diameter part of the agitating blade, and specifically, it can be calculated using the following formula.

```
Peripheral velocity v [m/s] = rω = 2×π×r[m]×f[rpm]/60
```

Here, r is the maximum radius of the agitating blade, $\omega$ is the angular velocity, f is the number of rotations of the agitating blade per unit of time, and n is a circular constant.

In the above description, the agitation energy is increased or decreased by changing the various conditions (agitation time, peripheral velocity of the agitating blade, temperature of the microparticle raw material solution) using the same agitator. However, the agitation energy may be increased or decreased by changing the above-mentioned various conditions using a plurality of apparatuses as long as a microparticle raw material solution in a dissolved state at the molecular level or molecular dispersion state is obtained and the properties/characterstics of the microparticles separated by mixing the microparticle raw material solution and the separating solvent in the thin film fluid can be controlled.

Further, when a microparticle raw material solution in a dissolved state at the molecular level or a molecular dispersion state can be obtained, the present invention can be carried out by changing the other conditions of the agitator. For example, there is a possibility that a microparticle raw material solution in a dissolved state at the molecular level or a molecular dispersion state can also be obtained by changing the combination of the shape of the blade 107 of the impeller 105 and the shape of the discharge port 125 of the screen 122 shown in FIG. 4 and FIG 5. More specifically, a microparticle raw material solution in a dissolved state at the molecular level or a molecular dispersion state can also be obtained by using and changing the combination of (A) a blade 107 extending in a curved manner in the opposite direction of the rotation direction of the rotation shaft 106 as it distances from the rotation shaft 106, (B) a blade 107 extending linearly in the radius direction of the rotation shaft, (C) a screen 122 having 24 discharge ports 125 each 1 mm in width, or (D) a screen 122 having 24 discharge ports 125 each 2 mm in width, and afterwards, the characteristics/properties of the microparticles separated by mixing the microparticle raw material solution and the separation solvent in the thin film fluid can be controlled.

For the above-mentioned combinations, when the microparticle raw material solution is high in concentration, it is considered that the agitation effect of the solvent is increased and the solubility and the dispersibility of the molecules and ions can be improved.

Hereinafter, an embodiment of the apparatus to separate the microparticles between at least two processing surfaces which are disposed in a position they are faced with each other so as to be able to approach to and separate from each other, at least one of which rotates relative to the other, will be explained by referring to the figures.

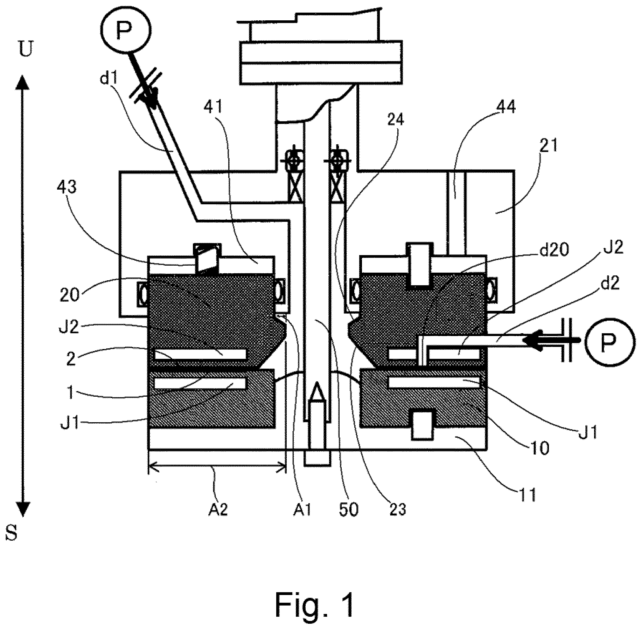

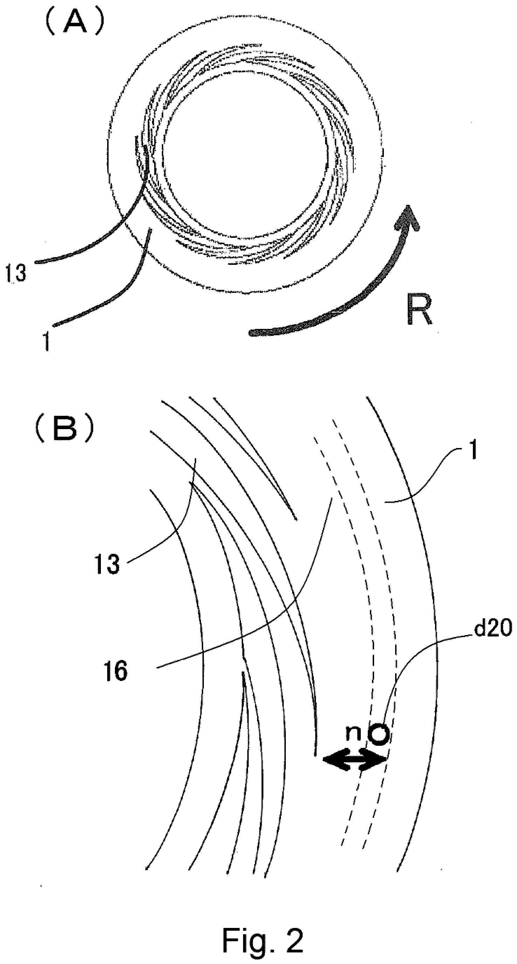

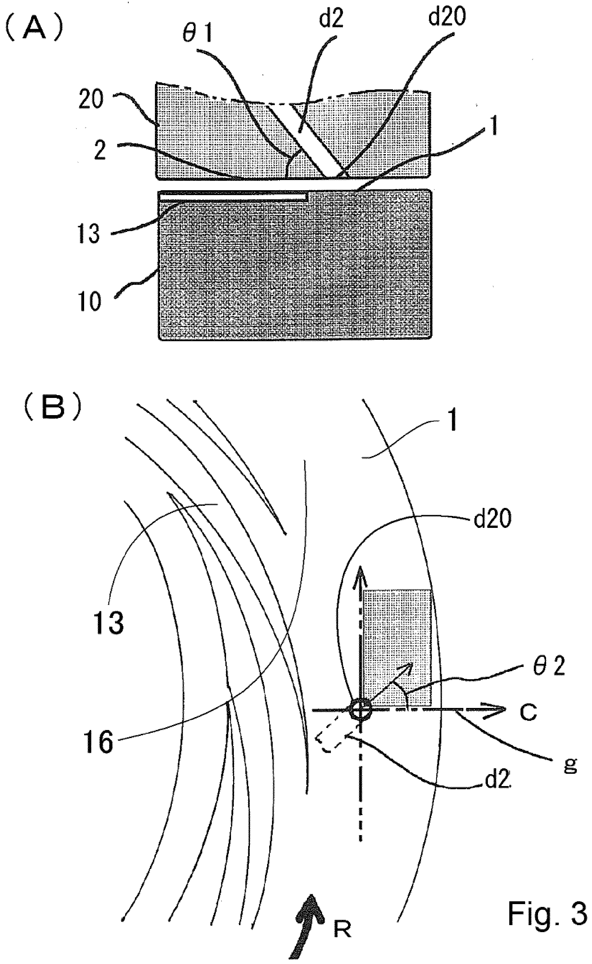

The fluid processing apparatus shown in FIG. 1 to FIG. 3 is similar to the apparatus described in Patent Document 3, with which a material to be processed is processed between processing surfaces in processing members arranged so as to be able to approach to and separate from each other, at least one of which rotates relative to the other; wherein, of the fluids to be processed, a first fluid to be processed, i.e., a first fluid, is introduced into between the processing surfaces, and a second fluid to be processed, i.e., a second fluid, is introduced into between the processing surfaces from a separate path that is independent of the flow path introducing the afore-mentioned fluid and has an opening leading to between the processing surfaces, whereby the first fluid and the second fluid are mixed and stirred between the processing surfaces. Meanwhile, in FIG. 1, a reference character U indicates an upside and a reference character S indicates a downside; however, up and down, front and back and right and left shown therein indicate merely a relative positional relationship and does not indicate an absolute position. In FIG. 2(A) and FIG. 3(B), reference character R indicates a rotational direction. In FIG. 3(B), reference character C indicates a direction of centrifugal force (a radial direction).

In this apparatus provided with processing surfaces arranged opposite to each other so as to be able to approach to and separate from each other, at least one of which rotates relative to the other, at least two kinds of fluids as fluids to be processed are used, wherein at least one fluid thereof contains at least one kind of material to be processed, a thin film fluid is formed by converging the respective fluids between these processing surfaces, and the material to be processed is processed in this thin film fluid. With this apparatus, a plurality of fluids to be processed may be processed as mentioned above; but a single fluid to be processed may be processed as well.

This fluid processing apparatus is provided with two processing members of a first processing member 10 and a second processing member 20 arranged opposite to each other, wherein at least one of these processing members rotates. The surfaces arranged opposite to each other of the respective processing members 10 and 20 are made to be the respective processing surfaces. The first processing member 10 is provided with a first processing surface 1 and the second processing member 20 is provided with a second processing surface 2.

The processing surfaces 1 and 2 are connected to a flow path of the fluid to be processed and constitute part of the flow path of the fluid to be processed. Distance between these processing surfaces 1 and 2 can be changed as appropriate; and thus, the distance thereof is controlled so as to form a minute space usually in the range of 1 mm or less, for example, about 0.1 $\mu$m to 50 $\mu$m. With this, the fluid to be processed passing through between the processing surfaces 1 and 2 becomes a forced thin film fluid forced by the processing surfaces 1 and 2.

When a plurality of fluids to be processed are processed by using this apparatus, the apparatus is connected to a flow path of the first fluid to be processed whereby forming part of the flow path of the first fluid to be processed; and part of the flow path of the second fluid to be processed other than the first fluid to be processed is formed. In this apparatus, the two paths converge into one, and two fluids to be processed are mixed between the processing surfaces 1 and 2 so that the fluids may be processed by reaction and so on. It is noted here that the term "process(ing)" includes not only the embodiment wherein a material to be processed is reacted but also the embodiment wherein a material to be processed is only mixed or dispersed without accompanying reaction.

To specifically explain, this apparatus is provided with a first holder 11 for holding the first processing member 10, a second holder 21 for holding the second processing member 20, a surface-approaching pressure imparting mechanism, a rotation drive mechanism, a first introduction part d1, a second introduction part d2, and a fluid pressure imparting mechanism p.

As shown in FIG. 2(A), in this embodiment, the first processing member 10 is a circular body, specifically a disk with a ring form. Similarly, the second processing member 20 is a circular disk. Material of the processing members 10 and 20 is not only metal but also ceramics, sintered metal, abrasion-resistant steel, sapphire, and other metal subjected to hardening treatment, and rigid material subjected to lining, coating, or plating. In the processing members 10 and 20 of this embodiment, at least part of the first and the second surfaces 1 and 2 arranged opposite to each other is mirror-polished.

Roughness of this mirror polished surface is not particularly limited; but surface roughness Ra is preferably 0.01 μm to 1.0 μm, or more preferably 0.03 μm to 0.3 μm.

At least one of the holders can rotate relative to the other holder by a rotation drive mechanism such as an electric motor (not shown in drawings). A reference numeral 50 in FIG. 1 indicates a rotary shaft of the rotation drive mechanism; in this embodiment, the first holder 11 attached to this rotary shaft 50 rotates, and thereby the first processing member 10 attached to this first holder 11 rotates relative to the second processing member 20. As a matter of course, the second processing member 20 may be made to rotate, or the both may be made to rotate. Further in this embodiment, the first and second holders 11 and 21 may be fixed, while the first and second processing members 10 and 20 may be made to rotate relative to the first and second holders 11 and 21.

At least any one of the first processing member 10 and the second processing member 20 is able to approach to and separate from at least any other member, thereby the processing surfaces 1 and 2 are able to approach to and separate from each other.

In this embodiment, the second processing member 20 approaches to and separates from the first processing member 10, wherein the second processing member 20 is accepted in an accepting part 41 arranged in the second holder 21 so as to be able to rise and set. However, as opposed to the above, the first processing member 10 may approach to and separate from the second processing member 20, or both the processing members 10 and 20 may approach to and separate from each other.

This accepting part 41 is a depression for mainly accepting that side of the second processing member 20 opposite to the second processing surface 2, and this depression is a groove being formed into a circle, i.e., a ring when viewed in a plane. This accepting part 41 accepts the second processing member 20 with sufficient clearance so that the second processing member 20 may rotate. Meanwhile, the second processing member 20 may be arranged so as to be movable only parallel to the axial direction; alternatively, the second processing member 20 may be made movable, by making this clearance larger, relative to the accepting part 41 so as to make the center line of the processing member 20 inclined, namely unparallel, to the axial direction of the accepting part 41, or movable so as to depart the center line of the processing member 20 and the center line of the accepting part 41 toward the radius direction.

It is preferable that the second processing member 20 be accepted by a floating mechanism so as to be movable in the three dimensional direction, as described above.

The fluids to be processed are introduced into between the processing surfaces 1 and 2 from the first introduction part d1 and the second introduction part d2 under the state that pressure is applied thereto by a fluid pressure imparting mechanism p consisting of various pumps, potential energy, and so on. In this embodiment, the first introduction part d1 is a path arranged in the center of the circular, second holder 21, and one end thereof is introduced into between the processing surfaces 1 and 2 from inside the circular, processing members 10 and 20. Through the second introduction part d2, the first fluid to be processed and the second fluid to be processed for reaction are introduced into between the processing surfaces 1 and 2. In this embodiment, the second introduction part d2 is a path arranged inside the second processing member 20, and one end thereof is open at the second processing surface 2. The first fluid to be processed which is pressurized with the fluid pressure imparting mechanism p is introduced from the first introduction part d1 to the space inside the processing members 10 and 20 so as to pass through between the first and second processing surfaces 1 and 2 to outside the processing members 10 and 20. From the second introduction part d2, the second fluid to be processed which is pressurized with the fluid pressure imparting mechanism p is provided into between the processing surfaces 1 and 2, whereat this fluid is converged with the first fluid to be processed, and there, various fluid processing such as mixing, stirring, emulsification, dispersion, reaction, deposition, crystallization, and separation are effected, and then the fluid thus processed is discharged from the processing surfaces 1 and 2 to outside the processing members 10 and 20. Meanwhile, an environment outside the processing members 10 and 20 may be made negative pressure by a vacuum pump.

The surface-approaching pressure imparting mechanism mentioned above supplies the processing members with force exerting in the direction of approaching the first processing surface 1 and the second processing surface 2 each other. In this embodiment, the surface-approaching pressure imparting mechanism is arranged in the second holder 21 and biases the second processing member 20 toward the first processing member 10.

The surface-approaching pressure imparting mechanism is a mechanism to generate force (hereinafter, surface-approaching pressure) to press the first processing surface 1 of the first processing member 10 and the second processing surface 2 of the second processing member 20 in the direction to make them approach to each other. The mechanism generates a thin film fluid having minute thickness in a level of nanometer or micrometer by the balance between the surface-approaching pressure and the force to separate the processing surfaces 1 and 2 from each other, i.e., the force such as the fluid pressure. In other words, the distance between the processing surfaces 1 and 2 is kept in a predetermined minute distance by the balance between these forces.

In the embodiment shown in FIG. 1, the surface-approaching pressure imparting mechanism is arranged between the accepting part 41 and the second processing member 20. Specifically, the surface-approaching pressure imparting mechanism is composed of a spring 43 to bias the second processing member 20 toward the first processing member 10 and a biasing-fluid introduction part 44 to introduce a biasing fluid such as air and oil, wherein the surface-approaching pressure is provided by the spring 43 and the fluid pressure of the biasing fluid. The surface-approaching pressure may be provided by either one of this spring 43 and the fluid pressure of this biasing fluid; and other forces such as magnetic force and gravitation may also be used. The second processing member 20 recedes from the first processing member 10 thereby making a minute space between the processing surfaces by separating force, caused by viscosity and the pressure of the fluid to be processed applied by the fluid pressure imparting mechanism p, against the bias of this surface-approaching pressure imparting mechanism. By this balance between the surface-approaching pressure and the separating force as mentioned above, the first processing surface 1 and the second processing surface 2 can be set with the precision of a micrometer level; and thus the minute space between the processing surfaces 1 and 2 may be set. The separating force mentioned above includes fluid pressure and viscosity of the fluid to be processed, centrifugal force by rotation of the processing members, negative pressure when negative pressure is applied to the biasing-fluid introduction part 44, and spring force when the spring 43 works as a pulling spring. This surface-approaching pressure imparting mechanism may be arranged also in the first processing member 10, in place of the second processing member 20, or in both the processing members.

To specifically explain the separation force, the second processing member 20 has the second processing surface 2 and a separation controlling surface 23 which is positioned inside the processing surface 2 (namely at the entering side of the fluid to be processed into between the first and second processing surfaces 1 and 2) and next to the second processing surface 2. In this embodiment, the separation controlling surface 23 is an inclined plane, but may be a horizontal plane. The pressure of the fluid to be processed acts to the separation controlling surface 23 to generate force directing to separate the second processing member 20 from the first processing member 10. Therefore, the second processing surface 2 and the separation controlling surface 23 constitute a pressure receiving surface to generate the separation force.

In the example shown in FIG. 1, an approach controlling surface 24 is formed in the second processing member 20. This approach controlling surface 24 is a plane opposite, in the axial direction, to the separation controlling surface 23 (upper plane in FIG. 1) and, by action of pressure applied to the fluid to be processed, generates force of approaching the second processing member 20 toward the first processing member 10.

Meanwhile, the pressure of the fluid to be processed exerted on the second processing surface 2 and the separation controlling surface 23, i.e., the fluid pressure, is understood as force constituting an opening force in a mechanical seal. The ratio (area ratio A1/A2) of a projected area A1 of the approach controlling surface 24 projected on a virtual plane perpendicular to the direction of approaching and separating the processing surfaces 1 and 2, that is, in the direction of rising and setting of the second processing member 20 (axial direction in FIG. 1), to a total area A2 of the projected area of the second processing surface 2 of the second processing member 20 and the separation controlling surface 23 projected on the virtual plane is called as balance ratio K, which is important for control of the opening force. This opening force can be controlled by the pressure of the fluid to be processed, i.e., the fluid pressure, by changing a balance line, i.e., by changing the area A1 of the approach controlling surface 24.

Sliding surface actual surface pressure P, i.e., the fluid pressure out of the surface-approaching pressures, is calculated according to the following equation:

$$P = P1 \times (K - k) + Ps$$

Here, P1 represents the pressure of a fluid to be processed, i.e., the fluid pressure, K represents the balance ratio, k represents an opening force coefficient, and Ps represents a spring and back pressure.

By controlling this balance line to control the sliding surface actual surface pressure P, the space between the processing surfaces 1 and 2 is formed as a desired minute space, thereby forming a fluid film of the fluid to be processed so as to make the processed substance such as a product fine and to effect uniform processing by reaction.

Meanwhile, the approach controlling surface 24 may have a larger area than the separation controlling surface 23, though this is not shown in the drawing.

The fluid to be processed becomes a forced thin film fluid by the processing surfaces 1 and 2 that keep the minute space therebetween, whereby the fluid is forced to move out from the circular, processing surfaces 1 and 2. However, the first processing member 10 is rotating; and thus, the mixed fluid to be processed does not move linearly from inside the circular, processing surfaces 1 and 2 to outside thereof, but does move spirally from the inside to the outside thereof by a resultant vector acting on the fluid to be processed, the vector being composed of a moving vector toward the radius direction of the circle and a moving vector toward the circumferential direction.

Meanwhile, a rotary shaft 50 is not only limited to be placed vertically, but may also be placed horizontally, or at a slant. This is because the fluid to be processed is processed in a minute space between the processing surfaces 1 and 2 so that the influence of gravity can be substantially eliminated. In addition, this surface-approaching pressure imparting mechanism can function as a buffer mechanism of micro-vibration and rotation alignment by concurrent use of the foregoing floating mechanism with which the second processing member 20 may be held displaceably.

In the first and second processing members 10 and 20, the temperature thereof may be controlled by cooling or heating at least any one of them; in FIG. 1, an embodiment having temperature regulating mechanisms J1 and J2 in the first and second processing members 10 and 20 is shown. Alternatively, the temperature may be regulated by cooling or heating the introducing fluid to be processed. These temperatures may be used to separate the processed substance or may be set so as to generate Benard convection or Marangoni convection in the fluid to be processed between the first and second processing surfaces 1 and 2.

As shown in FIG. 2, in the first processing surface 1 of the first processing member 10, a groove-like depression 13 extended toward an outer side from the central part of the first processing member 10, namely in a radius direction, may be formed. The depression 13 may be, as a plane view, curved or spirally extended on the first processing surface 1 as shown in FIG. 2(B), or, though not shown in the drawing, may be extended straight radially, or bent at a right angle, or jogged; and the depression may be continuous, intermittent, or branched. In addition, this depression 13 may be formed also on the second processing surface 2, or on both the first and second processing surfaces 1 and 2. By forming the depression 13 as mentioned above, the micro-pump effect can be obtained so that the fluid to be processed may be sucked into between the first and second processing surfaces 1 and 2.

It is desirable that the base end of this depression 13 reach the internal circumference of the first processing member 10. The front end of the depression 13 is extended toward the outer circumference side of the first processing surface 1, and the depth thereof (cross section area) becomes gradually shallower (smaller) as going from the base end to the front end.

Between the front end of the depression 13 and the outer circumference side of the first processing surface 1 is arranged the flat plane 16 not having the depression 13.

When an opening d20 of the second introduction part d2 is arranged in the second processing surface 2, the arrangement is done preferably at a position opposite to the flat surface 16 of the first processing surface 1 arranged at a position opposite thereto.

This opening d20 is arranged preferably in the downstream (outside in this case) of the depression 13 of the first processing surface 1. The opening is arranged especially preferably at a position opposite to the flat surface 16 located nearer to the outer diameter than a position where the direction of flow upon introduction by the micro-pump effect is changed to the direction of a spiral and laminar flow formed between the processing surfaces. Specifically, in FIG. 2(B), a distance n from the outermost side of the depression 13 arranged in the first processing surface 1 in the radial direction is preferably about 0.5 mm or more. Especially in the case of separating microparticles from a fluid, it is preferable that mixing of a plurality of fluids to be processed and separation of the microparticles therefrom be effected under the condition of a laminar flow.

The shape of the opening d20 may be circular as shown in FIG. 2 (B) and FIG. 3(B) or, although it is not illustrated, a concentric circular ring shape surrounding an opening at the center of a ring-shaped disc processing surface 2. Further, in cases where the opening is formed into a circular ring shape, the opening of the circular ring shape may be continuous or discontinuous.

When the circular ring shaped opening d20 is formed concentrically surrounding the opening at the center of the processing surface 2, the introduction of the second fluid into between the processing surfaces 1 and 2 can be carried out under the same conditions in the circumferential direction. Therefore, in the case of mass producing microparticles, it is preferable that the shape of the opening be formed into a concentric circular ring shape.

This second introduction part d2 may have directionality. For example, as shown in FIG. 3(A), the direction of introduction from the opening d20 of the second processing surface 2 is inclined at a predetermined elevation angle (θ1) relative to the second processing surface 2. The elevation angle (θ1) is set at more than 0° and less than 90°, and when the reaction speed is high, the angle (θ1) is preferably set in the range of 1° to 45°.

In addition, as shown in FIG. 3(B), introduction from the opening d20 of the second processing surface 2 has directionality in a plane along the second processing surface 2. The direction of introduction of this second fluid is in the outward direction departing from the center in a radial component of the processing surface and in the forward direction in a rotation component of the fluid between the rotating processing surfaces. In other words, a predetermined angle (θ2) exists facing the rotation direction R from a reference line g, which is the line to the outward direction and in the radial direction passing through the opening d20. This angle (θ2) is also set preferably at more than 0° and less than 90°.

This angle (θ2) can vary depending on various conditions such as the type of fluid, the reaction speed, viscosity, and the rotation speed of the processing surface. In addition, it is also possible not to give the directionality to the second introduction part d2 at all.

In the embodiment shown in FIG. 1, kinds of the fluid to be processed and numbers of the flow path thereof are set two respectively; but they may be one, or three or more. In the embodiment shown in FIG. 1, the second fluid is introduced into between the processing surfaces 1 and 2 from the introduction part d2; but this introduction part may be arranged in the first processing member 10 or in both. Alternatively, a plurality of introduction parts may be arranged relative to one fluid to be processed. The opening for introduction arranged in each processing member is not particularly restricted in its form, size, and number; and these may be changed as appropriate. The opening for introduction may be arranged just before the first and second processing surfaces 1 and 2 or in the side of further upstream thereof.

Meanwhile, because it is good enough only if the reaction could be effected between the processing surfaces 1 and 2, as opposed to the foregoing method, a method wherein the second fluid is introduced from the first introduction part d1 and a solution containing the first fluid is introduced from the second introduction part d2 may also be used. That is, the expression "first" or "second" for each fluid has a meaning for merely discriminating an $n^{th}$ fluid among a plurality of the fluids present; and therefore, a third or more fluids can also exist.

In the apparatus described above, the processing such as separation, precipitation, and crystallization takes place with forced and uniform mixing between the processing surfaces 1 and 2 which are disposed in a position they are faced with each other so as to be able to approach to and separate from each other, at least one of which rotates relative to the other, as shown in FIG. 1. Particle diameter and monodispersity of the substance to be processed that is so processed can be controlled by appropriately adjusting rotation speed of the processing members 10 and 20, flow velocity, distance between the processing surfaces 1 and 2, concentration of the raw material in the fluid to be processed, kind of the solvent for the fluid to be processed, and so on.

Hereinafter, specific embodiments of the method for producing the microparticles by using the apparatus described above will be explained.

In the above-mentioned apparatus, a microparticle raw material solution in which at least one type of microparticle raw material is dissolved in a solvent and at least one type of separating solution are introduced into between at least two processing surfaces, which are disposed in a position facing each other, so as to be able to approach to and separate from each other, at least one of which rotates relative to the other, and mixed in a thin film fluid formed between the at least two processing surfaces to thereby separate microparticles. At that time, the microparticle raw material solution is prepared using an agitator having rotation blades that rotate, and the agitation energy is increased or decreased by changing at least one of three conditions defining the agitation energy (agitation time, peripheral velocity of the agitating blade, and the temperature of the microparticle raw material solution).

The separation reaction of the microparticles takes place in the forced and uniform mixing between the processing surfaces 1 and 2 which are disposed in a position they are faced with each other so as to be able to approach to and separate from each other, at least one of which rotates relative to the other, in the apparatus shown in FIG. 1 of the present invention.

Firstly, at least one type of separating solvent is introduced as the first fluid from the first introduction part d1, which is one flow path, into between the processing surfaces 1 and 2 which are disposed in a position they are faced with each other so as to be able to approach to and separate from each other, at least one of which rotates relative to the other, thereby forming between the processing surfaces a first fluid film which is a thin film fluid formed of the first fluid.

Then, the microparticle raw material solution having at least one type of microparticle raw material dissolved in a solvent is introduced as the second fluid from the second introduction part d2, which is another flow path, directly into the first fluid film formed between the processing surfaces 1 and 2.

By so doing, the first fluid and the second fluid are mixed between the processing surfaces 1 and 2 while the distance therebetween is fixed by the pressure balance between the supply pressure of the fluids to be processed and the pressure that is applied between the rotating processing surfaces, thereby effecting the reaction to separate the microparticles.

Meanwhile, it is sufficient if the foregoing reaction can be effected between the processing surfaces 1 and 2; and thus, on contrary to the above, the second fluid may be introduced from the first introduction part d1, and the first fluid may be introduced from the second introduction part d2. In other words, the expression of the first and the second in each fluid merely distinguish the $n^{th}$ fluid among a plurality of fluids used therein; and thus, the third or more fluids may also exist.

Alternatively, as mentioned above, the processing apparatus may be provided with the third introduction part d3, in addition to the first introduction part d1 and the second introduction part d2; and in this case, for example, from the respective introduction parts, each of the first fluid, the second fluid, and the third fluid which is different from the first fluid and the second fluid may be introduced separately into the processing apparatus. By so doing, concentration and pressure of each fluid can be controlled separately so that the separation reaction can be controlled more precisely. Meanwhile, a combination of the fluids to be processed (first to third fluids) that are introduced into each of the introduction parts may be set arbitrarily. The same is applied if the fourth or more introduction parts are arranged; and by so doing, fluids to be introduced into the processing apparatus may be subdivided.

In addition, temperatures of the fluids to be processed such as the first fluid and the second fluid may be controlled; and temperature difference among the first fluid, the second fluid, and so on (namely, temperature difference among each of the supplied fluids to be processed) may be controlled either. To control temperature and temperature difference of each of the supplied fluids to be processed, a mechanism with which temperature of each of the fluids to be processed is measured (temperature of the fluid before introduction to the processing apparatus, or in more detail, just before introduction into between the processing surfaces 1 and 2) so that each of the fluids to be processed that is introduced into between the processing surfaces 1 and 2 may be heated or cooled may be installed.

The separation reaction of the pigment microparticles to be described later takes place with forced and uniform mixing between the processing surfaces 1 and 2 which are disposed in a position facing each other so as to be able to approach to and separate from each other, at least one of which rotates relative to the other. The control of the particle diameter and the monodispersity of the pigment microparticles, as well as the type of the crystal form can be adjusted by changing the number of rotations of the processing members 10 and 20, the flow velocity, the distance between the processing surfaces, the concentration of the raw material, and so on. As the applicant points out in Japanese Patent No. 4691698, etc., the applicant of this application puts emphasis on the separating step and worked on the production of microparticles having intended physical properties and functions by adjusting the rotation speed, the flow velocity, and the distance between the processing surfaces. However, the applicant found that, by adjusting the state of the dissolution of the fluid to be sent in the separating step by the adjustment of the dissolving step, especially the increase or decrease of the agitation energy, the degree of crystallinity and the crystal form of the microparticles obtained in the separating step can be controlled, and completed the present invention. With this, by merely changing the conditions in the dissolving step while fixing the conditions of the separating step, it becomes possible to change the degree of crystallinity and the crystal form of microparticles to thereby obtain intended properties and performance of microparticles, or by changing the conditions of both the dissolving step and the separating step, it becomes possible to change more dynamically the intended properties and performance of the microparticles.

For the separation reaction of the pigment microparticles, a variety of liquid-phase methods such as, e.g., an acid pasting method in which a pigment bulk powder is dissolved in a strong acid such as sulfuric acid, nitrate, and hydrochloric acid and a prepared pigment acidic solution is mixed with a solution including water or an organic solvent to obtain pigment microparticles, an alkaline pasting method in which a pigment bulk powder is dissolved in an alkaline solution and the prepared pigment alkaline solution is mixed with a solution including water or an organic solvent to obtain pigment microparticles, a redeposition method, a pH adjustment method, a poor solvent method, and the like.

These separation reactions can be carried out by conventionally well-known methods such as the one described in Patent Document 3, for example.

Hereinafter, the reaction that the pigment microparticles are generated using the above-mentioned apparatus is described in more detail.

(Acid Pasting Method)

When using the above-mentioned apparatus for an acid pasting method, firstly, a solution including water or an organic solvent is introduced as the first fluid from the first introduction part d1, which is one flow path, into between the processing surfaces 1 and 2 which are disposed in a position that they face each other so as to be able to approach to and separate from each other, at least one of which rotates relative to the other, thereby forming between the processing surfaces a thin film fluid formed by the first fluid.

Then, a fluid (pigment acidic solution) including an acid in which the pigment substance, which is a reactant, is dissolved is introduced as the second fluid from the second introduction part d2, which is another flow path, directly into the fluid film formed by the first fluid.

As described above, the first fluid and the second fluid are instantaneously mixed between the processing surfaces 1 and 2 in which the distance is fixed by the pressure balance between the supply pressure of the fluids and the pressure applied between the rotating processing surfaces and the first fluid and the second fluid while maintaining an ultrathin film state, thereby effecting the reaction that generates pigment microparticles.

Meanwhile, it is sufficient if the above-mentioned reaction can be effected between the processing surfaces 1 and 2; and thus, on the contrary to the above, the second fluid may be introduced from the first introduction part d1, and the first fluid may be introduced from the second introduction part d2. In other words, the expression of the first and the second in each solvent merely distinguish the $n^{th}$ solvent among a plurality of solvents; and thus, the third or more solvents may also exist.

As described above, the first fluid is a solution including water or an organic solvent. As the water, purified water such as ion-exchanged water, pure water, or distilled water is preferred. Further, an alkaline solution in which a solution including water or an organic solvent is alkalized can be used, and when the solution including water or an organic solvent is alkalized, aqueous ammonia, a sodium hydroxide aqueous solution, and a potassium hydroxide solution can be used as examples. Methanol, ethanol, and propanol may also be used.

An acid used for the second fluid is not particularly restricted as long as it shows solubility to pigments, but, for example, in the case of an acidic aqueous solution, sulfuric acid, hydrochloric acid, nitric acid, and trifluoroacetic acid may be used. Preferably, a strong acid, especially a concentrated sulfuric acid of 95% or more, can be used.

Further, an organic solvent may be mixed to the first fluid or the second fluid for the purpose of controlling the crystal form of the pigment or the quality control of the pigment. Well-known organic solvents may be used as the organic solvent. Furthermore, a dispersing agent such as a block copolymer, a high molecular polymer, and a surfactant may be included besides organic solvents.

(Redeposition Method)

Next, when using the above-mentioned apparatus for a redeposition method, firstly, a solvent that is a poor solvent to the pigment and compatible with the later-described solvent is introduced as the first fluid from the first introduction part d1, which is one flow path, into between the processing surfaces 1 and 2 which are disposed in a position that they face each other so as to be able to approach to and separate from each other, at least one of which rotates relative to the other, thereby forming between the processing surfaces 1 and 2 a thin film fluid formed by the first fluid.

Then, a fluid including an organic solvent in which pigment substances are dissolved is introduced as the second fluid from the second introduction part d2, which is another flow path, directly into the thin fluid film formed by the first fluid.

As described above, the first fluid and the second fluid are instantly mixed between the processing surfaces 1 and 2 while the distance therebetween is fixed by the pressure balance between the supply pressure of the fluids and the pressure applied between the processing surfaces 1 and 2 while maintaining an ultrathin film state, thereby effecting the reaction that generates pigment microparticles.

Meanwhile, it is sufficient if the foregoing reaction can be effected between the processing surfaces 1 and 2; and thus, on the contrary to the above, the second fluid may be introduced from the first introduction part d1, and the first fluid may be introduced from the second introduction part d2. In other words, the expression of the first and the second in each solvent merely distinguish the $n^{th}$ solvent among a plurality of solvents; and thus, the third or more solvents may also exist.

As described above, the first fluid is not particularly restricted as long as it is compatible with the solvent which is a poor solvent to pigments and dissolves the pigment forming the second fluid, but it is preferable to be chosen from the following or a mixed solvent of two or more among water, an alcohol-based solvent, a ketone-based solvent, an ether-based solvent, an aromatic-based solvent, a carbon disulfide, an aliphatic-based solvent, a nitrile-based solvent, a sulfoxide-based solvent, a halogen-based solvent, an ester-based solvent, and an ionic liquid.

The organic solvent used for the second fluid is not particularly restricted as long as it is soluble in pigments, but an amide-based solvent such as 1-methyl-2-pyrrolidone, 1,3-dimethyl-2-imidazolidinone, 2-pyrrolidinone, $\varepsilon$-caprolactam, formamide, N-methyl formamide, N,N-dimethylformamide, acetamide, N-methylacetamide, N,N-dimethylacetamide, N-methylpropanamide, hexamethylphosphoric triamide is preferably used.

Furthermore, a dispersing agent such as a block copolymer, a high molecular polymer, and a surfactant may be included in the first fluid or the second fluid.

(pH Adjustment Method)

Next, when using the above-mentioned apparatus for a pH adjustment method, firstly, a pigment separating solution for changing the pH is introduced as the first fluid from the first introduction part d1, which is one flow path, into between the rotating processing surfaces 1 and 2, thereby forming between the processing surfaces a thin film fluid formed by the first fluid.

Then, a pigment solution in which at least one type of pigment is dissolved in an acidic or alkaline pH adjustment solution or a mixed solution of the above-mentioned pH adjustment solution and an organic solvent is introduced as the second fluid from the second introduction part d2, which is another flow path, directly into the thin fluid film formed by the first fluid.

As described above, the first fluid and the second fluid are instantaneously mixed between the processing surfaces 1 and 2 while the distance therebetween is controlled by the pressure balance between the supply pressure of the fluids and the pressure applied between the rotating processing surfaces 1 and 2 and the first fluid and the second fluid while maintaining the thin film state, thereby effecting the reaction that generates pigment microparticles.

Specifically, for example, an organic pigment that barely dissolves in an organic solvent is added to an alkaline solution in which an alkaline substance is added to the organic solvent and dissolved as an organic pigment solution (second fluid), and the organic pigment solution is added to a pigment separating solution (first fluid) in which water, another organic solvent, an organic solvent not including the above-mentioned alkaline substance, or a solution including acid is used. With this, the pH of the organic pigment solution changes, and therefore the separation reaction of the pigment can be effected between the processing surfaces 1 and 2. In this case, the acid and the alkaline to be added may be selected to be added for dissolving or separating the pigment according to the type of pigment.

Meanwhile, it is sufficient if the above-mentioned reaction can be effected between the processing surfaces 1 and 2; and thus, on the contrary to the above, the second fluid may be introduced from the first introduction part d1, and the first fluid may be introduced from the second introduction part d2. In other words, the expression of the first and the second in each fluid merely distinguishes the $n^{th}$ solvent among a plurality of fluids; and therefore the third or more fluids may also exist.

As described above, the pigment separating solution as the first fluid is not particularly restricted as long as it is a solution capable of changing the pH of the pigment solution, is not soluble in a pigment intended for separation, or is less soluble in pigments than the solvent included in the pigment solution as the second fluid, but it is comprised of water, an organic solvent, or a mixture thereof. As the water, purified water such as ion-exchanged water, pure water, or distilled water is preferred. Although the organic solvent is not particularly restricted, examples include a monohydric alcohol-based solvent represented by methanol, ethanol, isopropanol, t-butanol, etc.; a polyhydric alcohol-based solvent represented by ethylene glycol, propylene glycol, diethylene glycol, polyethylene glycol, thiodiglycol, dithiodiglycol, 2-methyl-1,3-propanediol, 1,2,6-hexanetriol, acetylene glycol derivatives, glycerin, or trimethylopropane, etc.; an amide-based solvent such as 1-methyl-2-pyrrolidinone, 1,3-dimethyl-2-imidazolidinone, 2-pyrrolidinone, ε-caprolactam, formamide, N-methylformamide, N,N-dimethylformamide, acetamide, N-methylacetamide, N,N-dimethylacetamide, N-methyl propane amide, hexamethylphosphoric triamide, urea, tetramethylurea, etc.; lower polyhydric alcohol monoalkyl ether-based solvent such as ethylene glycol monomethyl (or ethyl) ether, diethylene glycol monomethyl (or ethyl) ether, triethylene glycol monoethyl (or butyl) ether, etc.; polyether-based solvent such as ethylene glycol dimethyl ether (monoglyme), diethylene glycol dimethyl ether (diglyme), triethylene glycol dimethyl ether (triglyme), etc.; sulfur-containing solvent such as sulfolane, dimethyl sulfoxide, 3-fulfolene, etc.; polyfunctional solvent such as diacetone alcohol, diethanolamine, etc.; carboxylic acid-based solvent such as acetic acid, maleic acid, docosahexaenoic acid, trichloroacetic acid, trifluoroacetic acid, etc.; sulfonic acid-based solvent such as methaneslfonic acid, trifluoromethanesulfonic acid, etc.; benzene-based solvent such as benzene, toluene, xylene, etc.

Furthermore, it can be carried out as an acidic or alkaline pH adjustment solution in which an acidic or alkaline pH adjustment substance is added in a solvent. The pH adjustment substance in that case is not particularly restricted, but when it is alkaline, it is an inorganic base such as lithium hydroxide, sodium hydroxide, potassium hydroxide, calcium hydroxide, and barium hydroxide, or an organic alkali such as trialkylamine, diazabicycloundecene, and metal alkoxide. In the case of acid, it is an inorganic acid such as formic acid, nitric acid, sulfuric acid, hydrochloric acid, and phosphoric acid, or an organic acid such as acetic acid, trifluoroacetic acid, oxalic acid, methanesulfonic acid, and trifluoromethanesulfonate. It can be carried out by adding them in a solid state or adding them as an aqueous solution or an organic solvent solution.

The same solvent as the first fluid may be used as the solvent used for the pigment solution as the second fluid. However, it is preferable to select a solvent with better solubility to the pigment than the solvent included in the first fluid. Also, the same substance as the first fluid may be added as the pH adjustment substance. It is preferable that the pH adjustment substance be selected so that the solubility of the second fluid with respect to the pigments is better than the solvent included in the first fluid.

Further, the mixed solution (pH adjustment solution) of the solvent and the pH adjustment substance included in the above-mentioned first fluid and the second fluid may be used in a solution state in which all of the substances are completely melted together or in a suspended state.

Further, an organic solvent may be mixed to the first fluid or the second fluid for the purpose of controlling the crystal form of the pigment or the quality control of the pigments. As the organic solvent, a well-known organic solvent may be used. Furthermore, a dispersing agent and a surfactant such as high molecular polymer, block copolymer, and the like, may be included besides an organic solvent.

The pigment used in each of the above-mentioned methods is not particularly restricted, but examples include an well-known organic pigment such as a polycyclic quinone-based pigment, a perylene-based pigment, an azo-type pigment, an indigo pigment, a quinacridone pigment, and a phthalocyanine pigment.

Further, the pigment includes a granular solid and a pigment such as a dye compound. An example of the pigment includes an inorganic achromatic pigment, and an organic or inorganic chromatic pigment. A colorless or light color pigment, a metallic luster pigment and so on may also be used. A newly synthesized pigment for the present invention may also be used. Hereinafter, specific examples of the pigment will be exemplified.

For a black pigment, for example, examples include the following. That is, Raven 1060, Raven 1080, Raven 1170, Raven 1200, Raven 1250, Raven 1255, Raven 1500, Raven 2000, Raven 3500, Raven 5250, Raven 5750, Raven 7000, Raven 5250, Raven 5750, Raven 7000, Raven 5000 ULTRA II, Raven 1190 ULTRAII (all are manufactured by Columbian Carbon Co.). Also included are: Black PearlsL, Mogul -L, Regal 400R, Regal 660R, Regal 330R, Monarch 800, Monarch 880, Monarch 900, Monarch 1000, Monarch 1300, Monarch 1400 (all are manufactured by Cabot Corp.). Furthermore, included are: Color Black FW1, Color Black FW2, Color Black FW200, Color Black 18, Color Back S160, Color Black S170, Special Black 4, Special Black 4A, Special Black 6, Printex 35, Printex U, Printex 140U, Printex V, Printex 140V (all are manufactured by Degussa Corp.). Also included are: No. 25, No. 33, No. 40, No. 47, No. 52, No. 900, No. 2300, MCF-88, MA600, MA7, MA8, MA100 (all are manufactured by Mitsubishi Chemical Corp.) and so on. However, it is not limited to the above.

For a cyan pigment, for example, examples include the following. That is, included are: C. I. Pigment Blue-1, C.I. Pigment Blue-2, C.I. Pigment Blue-3. Also included are: C. I. Pigment Blue-15, C.I. Pigment Blue-15:2, C.I. Pigment Blue-15:3, C.I. Pigment Blue-15:4. Further included are: C. I. Pigment Blue-16, C.I. Pigment Blue-22, C.I. Pigment Blue-60 and so on.

For a magenta pigment, for example, examples include the following. That is, included are: C. I. Pigment Red-5, C.I. Pigment Red-7, C.I. Pigment Red-12. Also included are: C. I. Pigment Red-48, C.I. Pigment Red-48:1, C.I. Pigment Red-57, C.I. Pigment Red-112. Also included are: C. I. Pigment Red-122, C.I. Pigment Red-123, C.I. Pigment Red-146, C.I. Pigment Red-168. Also included are: C. I. Pigment Red-184, C.I. Pigment Red-202, C.I. Pigment Red-207 and so on.

For a yellow pigment, for example, examples include the following. That is, included are: C. I. Pigment Yellow-12, C.I. Pigment Yellow -13, C.I. Pigment Yellow -14, C.I. Pigment Yellow -16. Also included are: C. I. Pigment Yellow-17, C.I. Pigment Yellow -74, C.I. Pigment Yellow -83, C.I. Pigment Yellow -93. Also included are: C. I. Pigment Yellow-95, C.I. Pigment Yellow -97, C.I. Pigment Yellow -98, C.I. Pigment Yellow -114. Also included are: C. I. Pigment Yellow-128, C.I. Pigment Yellow -129, C.I. Pigment Yellow -151, C.I. Pigment Yellow -154 and so on.

Furthermore, various pigments may be used in accordance with the intended color other than the above-mentioned black, cyan, magenta, and yellow pigments. Representative examples include a violet pigment such as Pigment Violet-23, a green pigment such as Pigment Green-7, an orange pigment such as Pigment Orange-43, but anything having color as a pigment may be used.

Further, in the present invention, dyes may be used in the same manner as a pigment. Examples include C.I. Solvent Blue, -33, -38, -42, -45, -53, -65, -67, -70, -104, -114, -115, -135. Also, examples include C.I. Solvent Red, -25, -31, -86, -92, -97, -118, -132, -160, -186, -187, -219. Also, examples include C.I. Solvent Yellow, -1, -49, -62, -74, -79, -82, -83, -89, -90, -120, -121, -151, -153, -154 and so on.

A water-soluble dye may also be used. Examples include: a direct dye such as C.I. Direct Black,-17, -19, -22, -32, -38, -51, -62, -71, -108, -146, -154 ; C.I. Direct Yellow, -12, -24, -26, -44, -86, -87, -98, -100, -130, -142; C.I. Direct Red, -1, -4, -13, -17, -23, -28, -31, -62, -79, -81, -83, -89, -227, -240, -242, -243; C.I. Direct Blue -6, -22, -25, -71, -78, -86, -90, -106, -199; C.I. Direct Orange, -34, -39, -44, -46, -60; C.I. Direct Violet, -47, -48; C.I. Direct Brown, -109; C.I. Direct Green, -59 and so on, an acid dye such as C.I. Acid Black, -2, -7, -24, -26, -31, -52, -63, -112, -118, -168, -172, -208; C.I. Acid Yellow, -11, -17, -23, -25, -29, -42, -49, -61, -71; C.I. Acid Red,-1, -6, -8, -32, -37, -51, -52, -80, -85, -87, -92, -94, -115, -180, -254, -256, -289, -315, -317; C.I. Acid Blue, -9, -22, -40, -59, -93, -102, -104, -113, -117, -120, -167, -229, -234, -254; C.I. Acid Orange, -7, -19; C.I. Acid Violet, -49 and so on, a reactive dye such as C.I. Reactive Black, -1, -5, -8, -13, -14, -23, -31, -34, -39; C.I. Reactive Yellow, -2, -3, -13, -15, -17, -18, -23, -24, -37, -42, -57, -58, -64, -75, -76, -77, -79, -81, -84, -85, -87, -88, -91, -92, -93, -95, -102, -111, -115, -116, -130, -131, -132, -133, -135, -137, -139, -140, -142, -143, -144, -145, -146, -147, -148, -151, -162, -163; C.I. Reactive Red,-3, -13, -16, -21, -22, -23, -24, -29, -31, -33, -35, -45, -49, -55, -63, -85, -106, -109, -111, -112, -113, -114, -118, -126, -128, -130, -131, -141, -151, -170, -171, -174, -176, -177, -183, -184, -186, -187, -188, -190, -193, -194, -195, -196, -200, -201, -202, -204, -206, -218, -221; C.I. Reactive Blue, -2, -3, -5, -8, -10, -13, -14, -15, -18, -19, -21, -25, -27, -28, -38, -39, -40, -41, -49, -52, -63, -71, -72, -74, -75, -77, -78, -79, -89, -100, -101, -104, -105, -119, -122, -147, -158, -160, -162, -166, -169, -170, -171, -172, -173, -174, -176, -179, -184, -190, -191, -194, -195, -198, -204, -211, -216, -217; C. I. Reactive Orange,-5, -7, -11, -12, -13, -15, -16, -35, -45, -46, -56, -62, -70, -72, -74, -82, -84, -87, -91, -92, -93, -95, -97, -99; C.I. Reactive Violet, -1, -4, -5, -6, -22, -24, -33, -36, -38; C. I. Reactive Green, -5, -8, -12, -15, -19, -23; C.I. Reactive Brown, -2, -7, -8, -9, -11, -16, -17, -18, -21, -24, -26, -31, -32, -33 and so on; C.I. Basic Black, -2; C.I. Basic Red, -1, -2, -9, -12, -13, -14, -27; C.I. Basic Blue, -1, -3, -5, -7, -9, -24, -25, -26, -28, -29; C.I. Basic Violet, -7, -14, -27; C.I. Food black, -1, -2 and so on.

The dye to be used may be a well-known or new die. For example, a direct dye, an acid dye, a basic dye, a reactive dye, a water-soluble dye of food pigments, a fat-soluble (oil-soluble) dye, or an insoluble pigment of disperse dyes may be used. They can be used in a solidified state. From this point, an oil-soluble dye may be preferably used, for example.

The oil-soluble dye in the present invention refers to a dye that can be dissolved in an organic solvent, and is also referred to as a fat-soluble dye.

Various commercially available products used for dispersing pigments may be used as surfactants and dispersing agents. Although it is not particularly restricted, examples include: dodecylbenzenesulfonic acids such as NEOGEN R-K (manufactured by Dai-ichi Kogyo Seiyaku Co. Ltd.); Solsperse 20000, Solsperse 24000, Solsperse 26000, Solsperse 27000, Solsperse 28000, Solsperse 41090 (manufactured by Avecia, Co. Ltd.); DISPERBYK-160, DISPERBYK-161, DISPERBYK-162, DISPERBYK-163, DISPERBYK-166, DISPERBYK-170, DISPERBYK-180, DISPERBYK-181, DISPERBYK-182, DISPERBYK-183, DISPERBYK-184, DISPERBYK-190, DISPERBYK-191, DISPERBYK-192, DISPERBYK-2000, DISPERBYK-2001 (manufactured by BYK-Chemie, Co. Ltd.) ; polymer 100, polymer 120, polymer150, polymer 400, polymer 401, polymer 402, polymer 403, polymer 450, polymer 451, polymer 452, polymer 453, EFKA-46, EFKA-47, EFKA-48, EFKA-49, EFKA-1501, EFKA-1502, EFKA-4540, EFKA-4550, (manufactured by EF KA Chemical Co. Ltd.) ; FIOWLEN DOPA-158, FIOWLEN DOPA-22, FIOWLEN DOPA-17, FIOWLEN G-700, FIOWLEN TG-720W, FIOWLEN-730W, FIOWLEN-740W, FIOWLEN-745W (manufactured by Kyoeisha Chemical Co. LTD.) ; AJISPER PA111, AJISPER PB711, AJISPER PB811, AJISPER PB821, AJISPER PW911 (manufactured by Ajinomoto Co. Inc.); Joncryl 678, Joncryl 679, Joncryl 62 (manufactured by Johnson Polymers Ltd.) and so on. They may be used separately or two or more types may be combined.

As the block copolymer in the present invention, specific examples include the following. That is, an acrylic-based block copolymer, a methacrylic-based block copolymer, polystyrene and other addition-polymerized-based or condensation polymerization-based block copolymer, a block copolymer having a block polyoxyethylene and polyoxyalkylene, and so on. Also, a conventionally known block copolymer may be used. The block copolymer used in the present invention is preferably amphiphilic. Specifically, an example of a preferred form is a diblock copolymer comprised of a hydrophobic segment and a hydrophilic segment having organic acid or its ionic salt unit. Also, a triblock copolymer having a hydrophobic segment, a hydrophilic segment having organic acid or its ionic salt unit, and another segment may be preferably used. In the case of triblock, a hydrophobic segment, a nonionic hydrophilic segment, and a hydrophilic segment having organic acid or its ionic salt unit forms are preferably used, and also preferable for the stability of the contained state. For example, when a disperse solution is prepared using a pigment substance and water as a solvent by using the above-mentioned triblock copolymer, it is possible to contain the pigment in the micelle formed by the triblock copolymer, thereby making it possible to form a pigment-containing ink composition in that manner. Further, the particle diameter of the particles of the dispersion composition can be made extremely uniform and even. Furthermore, it is possible to make the dispersed state extremely stable. When these processes are performed using the above-mentioned apparatus, the evenness of the particle diameter of the pigment particles further improves extremely uniformly.

Further, as a method for producing pigment particles using the above-mentioned apparatus, other than the above-mentioned methods, a pigment may be synthesized directly in a thin film fluid. As an example, in the case of a copper phthalocyanine synthesis example, as represented by a method in which phthalic anhydride or its derivative, copper or its compound, urea or its derivative, and catalyst are reacted in an organic solvent or in the absence thereof to obtain a copper phthalocyanine pigment, a pigment may be directly synthesized utilizing various reactions. By so doing, necessary pulverizing of coarse pigment particles produced in the synthesizing step is no longer needed, and further, when the pulverizing step is necessary, a shearing force can be applied in a thin film fluid by the operating conditions to include the pulverizing step.

In the present invention, the mixing in the mixing flow path can be performed under the condition of a laminar flow or a turbulent flow.

Further, the space between the processing surfaces can be heated and cooled or irradiated with microwave. Furthermore, ultraviolet rays (UV) can be irradiated between the processing surfaces, or ultrasonic energy can be applied between the processing surfaces. Particularly, when a temperature difference is set between the first processing surface 1 and the second processing surface 2, a convection can be generated in the thin film fluid and there is an advantage that reactions can be facilitated.

More specifically, in heating and cooling, for example, by providing a heater or a jacket that allows a heating medium or a coolant to pass through for at least one or both of the processing members 10 and 20, for example, the thin film fluid can be heated or cooled. Alternatively, the heating and the reaction of the processing fluid can be facilitated by providing a microwave generating apparatus such as a magnetron for irradiating microwaves to at least one or both of the processing members 10 and 20. Furthermore, for irradiating ultraviolet rays (UV), for example, an element such as a lamp for irradiating ultraviolet rays to at least one or both of the processing members 10 and 20 can be provided so that ultraviolet rays (UV) can be irradiated to the thin film fluid from the corresponding processing surface. Also, the application of ultrasonic energy can be carried out by, for example, providing an ultrasonic vibrator in at least one or both of the processing members 10 and 20, and the mixing and the reaction between the processing surfaces can be performed in an ultrasonic atmosphere in a container.

Further, by performing the above-mentioned separation in a container capable of ensuring a reduced pressure or a vacuum state to thereby make at least the secondary side to which the fluid is discharged after the process into a reduced pressure or a vacuum state, the degassing of the gas generated during the separation reaction and the gas included in the fluid, as well as the desolvation of the above-mentioned fluid can be performed. By doing so, even when the desolvation process is performed approximately at the same time as the pigment particle separation, since the fluid including the pigment particles separated between the processing surfaces are discharged from the processing surfaces in a spray state, the surface area of the fluid increases, thereby making the efficiency of the desolvation extremely high. Therefore, the pigment particle producing process and the desolvation process can be performed essentially in one step more easily than before.

As described above, the processing apparatus may be provided with the third introduction part d3, in addition to the first introduction part d1 and the second introduction part d2. In this case, however, for example, in the above-mentioned acid pasting method, from the respective introduction parts, a solution including water or an organic solvent, a fluid including acid in which pigments are dissolved, an organic solvent for controlling the crystal form of pigments or controlling the quality of pigments, and so on, may be introduced separately into the processing apparatus. Further, in the case of adjusting the pH, a pigment separating solution for changing the pH, a fluid including a pigment solution, an organic solvent for controlling the crystal form of the pigments or controlling the quality of pigments, and so on, may be introduced separately into the processing apparatus from each of the introduction parts. By doing so, the concentration and the pressure of each of the solutions can be controlled separately so that the reaction generated by the pigment particles can be controlled more precisely. The same is applied if the fourth or more introduction parts are arranged; and by doing so, fluids to be introduced into the processing apparatus can be subdivided.

In the above-mentioned apparatus used in the present invention, the Reynolds number in its thin film fluid can be freely changed, and therefore, monodisperse pigment particles having excellent redispersibility can be produced in accordance with the intended purpose, such as the particle diameter, particle shape, and crystal form. Further due to its self-discharge property, there is no blockage of the product even in the case of a reaction accompanying separation, no big pressure is needed. Therefore, pigment particles can be produced stably, which are excellent in safety, hardly have mixed impurities, and excellent in washability. Furthermore, since it can be up-scaled according to the intended production amount, a method of producing pigment particles high in productivity can also be provided.

The separation reaction of the biological ingesting microparticles to be described next takes place in the forced and uniform mixing between the processing surfaces 1 and 2 which are disposed in a position they are faced with each other so as to be able to approach to and separate from each other, at least one of which rotates relative to the other. The control of the particle diameter, the monodispersity, or the crystal form of the biological ingesting microparticles can be adjusted by changing the rotation speed of the processing surfaces 1 and 2, the distance between the processing surfaces 1 and 2, the flow velocity, the temperature, or the concentration of the raw material in the thin film fluid, and so on. This is as pointed out by the applicant of the present invention in Japanese Patent No. 4419157 and so on. The applicant of the present invention put emphasis on the separating step by adjusting the rotation speed, the flow velocity, and the distance between the processing surfaces to produce microparticles having the intended properties and functions . However, it was found that the degree of crystallinity and the crystal form of the microparticles to be obtained in the separating step can be controlled by adjusting the dissolved state of the fluid to be sent in the separating step by increasing and decreasing the agitation energy, and the present invention was completed. With this, only by changing the conditions of the dissolving step while the conditions of the separating step is fixed, it becomes possible to change the degree of crystallinity and the crystal form of the microparticles to thereby obtain the intended properties and performance of the microparticles, or by changing both conditions in the dissolving step and the separating step, it becomes possible to more dynamically change the intended properties and performance of the microparticles.

Hereinafter, specific embodiments of the method for producing the biological ingesting microparticles by using the above-mentioned apparatus will be explained. Here, a method for separating the biological ingesting microparticles by the change of the solubility will be explained, but it may also be a method for separating biological ingesting materials are separated by a neutralization reaction or a pH change.

In the thin film fluid formed between the processing surfaces of the above-described apparatus, a solution including a first solvent in which at least one type of biological ingesting microparticle material, which is a substance subject to be micronized, is dissolved, and a solvent which becomes a second solvent lower in solubility than the first solvent with respect to the above-mentioned biological ingesting microparticle material are mixed to separate biological ingesting microparticles.

The above-mentioned biological ingesting material includes a medical substance. The present invention can be carried out for various medical substances. The medical substance is preferably an organic substance existing in an essentially pure state. The medical substance needs to be dispersible with a low solubility to at least one type of solvent and soluble in at least one type of solvent. "Low solubility" refers to solubility of the medical substance of less than about 10 mg/mL preferably about 1 mg/mL in a solvent (for example, water) at a processing temperature (for example, room temperature). Further, here, "soluble" refers to dissolving in 10 mg/mL or more. Also, the solvent may be heated or cooled according to need. Furthermore, a dispersant agent (surfactant), a water-soluble polymer, a stabilizer, a preservative, a pH adjuster, a tonicity agent, and so on, can be added to the first solvent or the second solvent, or both of them in advance as needed.

A suitable medicine can be selected from various known medicines, examples including: analgesic, anti-inflammatory drug, anthelmintic, antiarrhythmic drug, antibiotics (including penicillins), anticoagulant, antihypertensive, hypoglycemic drug, antiepileptic drug, antihistamine, antitumor drug, anti-obesity drug, appetite-suppressant, hypertensive, antimuscarinic drug, antimycobacterial drug, antineoplastic drug, immunosuppressant, antithyroid drug, antibacterial drug, antiviral drug, anxiolytic (hypnotic, neuroleptic), astringent, β-adrenoceptor blocker, blood products and plasma substitute, myocardial degeneration force drugs, contrast medium, corticosteroid, cough suppressant, (expectorant and mucolytic agent), diagnostic drug, diagnostic imaging agent, diuretic, dopamine agonist (antiparkinson drug), hemostatic agent, immunologic agent, lipid regulation agent, muscle-relaxant drug, parasympathomimetic drug, parathyroid calcitonin and bisphosphonates, prostaglandin, radiopharmaceutical, sexogen (including steroids), antiallergic agent, stimulant and appetite inhibitor, sympathomimetic drug, thyroid drug, vasodilator and xanthines, cataract agent, adrenocorticosteroid. An example of a preferable medicine includes medicines intended for oral administration or injecting having low solubility to water. The descriptions of the drugs in these classes and the list of the drugs included in each of these classes can be found in "Martindale, The Extra Pharmacopoeia, Edition No. 29, The Pharmaceutical Press, London, 1989." These medicines are sold commercially and can be produced with well-known methods in the present field of technology.

Specific examples of useful drugs for carrying out the present invention can be represented by: 17-α-pregna-2,4-diene-20-yne-[2,3-d]-isoxazol-17-ol (danazol), tacrolimus hydrate, progesterone, tranilast, benzbromarone, mefenamic acid, [6-methoxy-4-(1-methylethyl)-3-oxo-1,2-benzisothiazol-2(3H)-yellow] methyl 2,6-dichlorobenzoate 1,1-dioxide (WIN63, 394), 3-amino-1,2,4-benzotriazine-1,4-dioxide(WIN59,075), piposaru pham, piposaru fan, camptothecin, acetaminophen, acetylsalicylic acid, amiodarone, koresuchifumin, colestipol, cromolyn sodium, albuterol, sucralfate, sulphasalazine, minoxidil, tenpazepamu, alprazolam, propoxyphene, auranofin, erythromycin, cyclosporin, acyclovir, ganciclovir, etoposide, mephalan, methotrexate, minoki san tron, daunorubicin, doxorubicin, megestrol, tamoxifen, medroxyprogesterone, nystatin, terbutaline, amphotericinB, aspirin, ibuprofen, naproxen, indomethacin, diclofenac, ketoprofen, flurbiprofen, diflunisal, etheyl-3,5-diacetamide-2,4,6-tri-iodinated benzoate (WIN8883), ethyl (3,5-bis(acetylamino)-2,4,6-tri-iodinated benzoyloxy)acetate (WIN12,901) and ethyl-2-(3,5-bis(acetylamino)-2,4,6-tri-iodinated benzoyloxy) acetate (Win 16, 318).

In this preferred embodiment of the present invention, the medicine is an immunosuppressant such as Danazol or tacrolimus hydrate, an antiallergic drug such as tranilast, a steroid such as progesterone, an antiviral drug, an antineoplastic drug or an anti-inflammatory drug.

As particularly preferable stabilizer/dispersant (surfactant), dodecylbenzenesulfonate, sodium dodecyl sulfate, sodium tetradecyl sulfate, sodium pentadecyl sulfate, sodium octyl sulfate, sodium oleate, sodium lauryl sulfate, sodium stearate, calcium stearate, Tween20 and Tween80 (they are polyoxyethylene sorbitan fatty acid esters that can be obtained from ICI Specialty Chemicals), polyvinylpyrrolidone, tyloxapol, Pluronic F68 and 108 (they are ethylene oxide and propylene oxide block copolymers that can be obtained from BASF), Tetronic 908 (T908) (this is a quad-functional block copolymer derived from ethylene oxide and propylene oxide continuous adducts to ethylenediamine that can be obtained from BASF), dextran, lecithin, aerosol OT (ester of a sodium sulfosuccinate that can be obtained from American Cyanamid), Duponol P (this is a sodium lauryl sulfate that can be obtained from DuPont), Triton X-200 (this is an alkyl aryl polyester sulfonate that can be obtained from Rohm and Haas), Carbowax 3350 and 934 (they are polyethylene glycols that can be obtained from Union Carbide), Crodesta F-110 (this is a mixture of sucrose stearate and sucrose distearate that can be obtained from Croda Inc.), Crodesta 5L-40 (this can be obtained from Croda Inc.), and SA90HCO [this is $C_{18}H_{37}CH_2$- $(CON(CH_3)CH_2(CHOH)_4CH_2OH)_2$] as well as quaternary amine surfactant such as benzethonium chloride, benzalkonium chloride, and so on, and non-ionic surfactants such as polyoxyethylene higher alcohol ethers, glycerin-fatty acid esters, polyoxyethylene hardened castor oil, polyoxyethylene fatty acid ester, polyoxyethylene nonylphenyl ether, polyoxyethylene octyl phenyl ether, sorbitan fatty acid ester, propylene glycol-fatty acid ester, fatty acid-polyethylene glycol, polyglyceryl fatty acid ester, sucrose fatty acid esters. They can be used differently according to the intended biological ingesting microparticles and separating reaction.

As a water-soluble polymer, examples include methyl cellulose, ethyl cellulose, propyl methyl cellulose, propyl cellulose, carboxymethyl-cellulose, polyvinyl alcohol, polyvinylpyrrolidone, and so on. There are no particular restrictions for the content amount of the drugs in this present invention. A highly concentrated suspension can be made and diluted to be used as formulation according to the concentration of use.

As a stabilizer, examples include disodium edetate, sodium sulfite, sodium bisulfite, sodium thiosulfate, dibutylhydroxytoluene, tocopherol, and so on.

As a preservative, examples include p-hydroxybenzoate ester, chlorobutanol, phenylethyl alcohol, benzalkonium chloride, benzethonium chloride, chlorhexidine gluconate, alkylpolyaminoethylglycines, sorbic acid, and so on.

As a pH regulating agent, examples include hydrochloric acid, sulfuric acid, acetic acid, lactic acid, citric acid, tartaric acid, malic acid, phosphoric acid, boric acid, sodium hydroxide, potassium hydroxide, calcium hydroxide, monoethanolamine, diethanolamine, diethylamine, ammonia, and their salts.

As the tonicity agent, examples include sodium chloride, potassium chloride, calcium chloride, mannitol, and so on.

As a solvent used for a fluid including at least one type of the biological ingesting microparticle material in the present invention, water such as ultrapure water or ion-exchanged water, and a water-miscible organic solvent such as methyl alcohol, ethyl alcohol, acetone, dimethylformamide, dimethylacetamide, and dimethyl sulfoxide, and a water-immiscible organic solvent such as an octane, cyclohexane, benzene, xylene, diethyl ether, and ethyl acetate can be selected as appropriate in accordance with the intended purpose.

Further, the biological ingesting microparticles of the present invention is not particularly restricted as long as it is intended to be biologically ingested, but examples include microparticles intended to be absorbed into the body such as pharmaceutical drugs to produce the effect in the body, microparticles that passes through the body such as a barium sulfate as a contrast medium, a transportation material of drug ingredients in a drug delivery system, microparticles to be applied to the skin of a body such as cosmetics, and an intermediate for a food product and the above-mentioned substances.

The separating reaction of the microparticles takes place with the forced and uniform mixing in the apparatus as shown in Fig. 1 between the processing surfaces 1 and 2 which are disposed in a position they are faced with each other so as to be able to approach to and separate from each other, at least one of which rotates relative to the other.

First, a solution including the above-mentioned first solvent is introduced from the first introduction part d1, which is one flow path, into between the processing surfaces 1 and 2 which are disposed in a position they are faced with each other so as to be able to approach to and separate from each other, at least one of which rotates relative to the other, thereby forming between the processing surfaces a thin film fluid which is a thin film fluid formed of the first fluid.

Next, a solvent as a second solvent having a lower solubility than the first solvent is introduced from the second introduction part d2, which is another flow path, directly into the thin film fluid formed of the first fluid.

As described above, a solution including the first solvent and the second solvent are mixed between the processing surfaces 1 and 2 while the distance therebetween is fixed by the pressure balance between the supply pressure of the fluids and the pressure that is applied between the rotating processing surfaces, thereby effecting the reaction to separate the microparticles.

Meanwhile, it is sufficient if the foregoing reaction can be effected between the processing surfaces 1 and 2; and thus, on contrary to the above, the second solvent may be introduced from the first introduction part d1, and the first solvent may be introduced from the second introduction part d2. In other words, the expression of the first and the second in each solvent merely distinguishes the $n^{th}$ fluid among a plurality of solvents used therein; and thus, the third or more fluids may also exist.

As described above, a third introduction part d3 can be provided in the processing apparatus besides the first introduction part d1 and the second introduction part d2, but in that case, for example, a solution including the first solvent, the second solvent, and a solution including a stabilizer and a dispersant can be introduced separately to the processing apparatus from each of the introduction parts. By doing so, concentration and pressure of each of the solutions can be controlled separately, so that the separating reaction can be controlled more precisely. The same is applied if the fourth or more introduction parts are arranged; and by doing so, fluids to be introduced into the processing apparatus may be subdivided.

EXAMPLES

Hereinafter, the present invention will be explained in more detail by Examples; but the present invention is not limited only to these Examples.

Meanwhile, in the following Examples, the term "from the center" means "from the first introduction part d1" of the processing apparatus shown in FIG. 1; the first fluid means the above-mentioned first fluid to be processed which is introduced from the first introduction part d1; and the second fluid means the above-mentioned second fluid to be processed which is introduced from the second introduction part d2 of the processing apparatus shown in FIG. 1. Further, as the opening d20 of the second introduction part d2, as shown by the dot-line in FIG. 2 (B), a concentric circular annular ring shaped opening surrounding the central opening of the processing surface 2 was used.

For a TEM observation, using JEM-2100 manufactured by Nippon Electronics Co., Ltd., the primary particle diameter was observed for plural visual fields. For the observation condition of the TEM observation, in Examples 1 to 40, the observation magnification was set to 50, 000 or more, and the average value of the primary particle diameter of 100 microparticles observed by the TEM observation was employed as an average particle diameter; and in Examples 41 to 49, the observation magnification was set to 30,000 or more, and the average value of the primary particle diameter of 100 microparticles observed by the TEM observation was employed as an average particle diameter.

For the X-ray diffraction (XRD) measurement, a powder X-ray diffraction measurement device X 'Pert PRO MPD (manufactured by XRD Spectris PANalytical Business Unit) was used.

As for Examples 1 to 40, the measurement conditions were Cu anticathode, tube voltage of 45 kV, tube current of 40 mA, 0.016 step/10 sec., measurement range of 10 to 60 [° 2Theta](Cu). The degree of crystallinity of the obtained microparticles and the component ratio of the particular crystal form were calculated from the XRD measurement results. The degree of crystallinity was calculated from the XRD measurement results obtained in each experiment by a constant background method assuming that the degree of crystallinity of the pigment bulk powder is 100%. The component ratio of the particular crystal form of β-type crystal (hereinafter referred to as "β-type crystal ratio) was calculated by the following formula (4) from the measurement results using the peak strength Iβ near 27.5° appeared as a characteristic peak in the β-type crystal and the peak strength Iα near 26.5° appeared as a characteristic peak in the α-type crystal.

$$\beta\text{-type crystal ratio}=(I\beta/(I\alpha+I\beta)\times100\ [\%] \qquad \text{Formula (4)}$$

Further, as for Examples 41 to 49, the measurement conditions were Cu anticathode, tube voltage of 45 kV, tube current of 40 mA, 0.016 step/10 sec., measurement range of 10 to 45 [° 2Theta] (Cu). The component ratio of the degree of crystallinity and the particular crystal form of the obtained microparticles was calculated from the XRD measurement results. The degree of crystallinity was calculated from the XRD measurement results obtained in each experiment by a constant background method assuming that the degree of crystallinity of the pigment bulk powder is 100%. The component ratio of the particular crystal form of γ-type crystal (hereinafter referred to as "γ-type crystal ratio) was calculated by the following formula (5) from the measurement results using the peak strength Iγ near 29.5° appeared as a characteristic peak in the γ-type crystal, the peak strength Iα near 15.5° appeared as a characteristic peak in the α-type crystal, and the peak strength Iβ near 10.5° appeared as a characteristic peak in the β-type crystal.

$$\gamma\text{-type crystal ratio}=(I\gamma/(I\alpha+I\beta)\times100\ [\%] \qquad \text{Formula (5)}$$

(Production of PR122 microparticles using an acid pasting method)

Using a fluid processing apparatus shown in Fig. 1, a pigment solution as a microparticle raw material solution and a separating solution were mixed in a thin film fluid formed between processing surfaces 1 and 2, which are arranged so as to face each other and provided with processing surfaces capable of approaching and separating with each other, at least one of the processing surfaces being capable of rotating with respect to the other. Thus, pigment microparticles were separated in the thin film fluid. The preparation of the pigment solution was performed using a rotating agitator having agitating blades, and the agitation energy was increased and decreased by changing at least one of three conditions deciding the agitation energy (the agitation time, the peripheral velocity of the agitating blade, the temperature of the microparticle raw material solution) . In Examples 1 to 3, the agitation energy was increased and decreased by changing the temperature (preparation temperature) of the pigment solution.
Initially, using the agitator (CLEARMIX (manufactured by M Technique Co., Ltd.)) having rorating agitating blades shown in FIG. 4 and FIG. 5, a pigment solution was prepared. Specifically, using CLEARMIX, PR122 was added to 10 wt% fuming sulfuric acid so that the total amount becomes 3 wt%, the pigment solution was agitated in a nitrogen atmosphere according to the preparation time, the preparation temperature, the peripheral velocity of the agitating blade shown in Table 2.
Next, while supplying methanol (MeOH) as a separating solution of the first fluid from the center at the supply pressure/back pressure = 0.121 MPaG/0.020 MPaGm and the number of rotations of 2500 rpm, the pigment solution was introduced into between the processing surfaces as a second fluid, and the first fluid and the second fluid was mixed in the thin film fluid. PR122 microparticle dispersion liquid was discharged from between the processing surfaces 1 and 2. In order to remove the impurities from the discharged PR122 microparticle dispersion liquid, the PR122 microparticle dispersion liquid was softly agglutinated, and the PR122 microparticle dispersion liquid was allowed to be settled by a centrifugal separator (x18000G) as a cleansing operation. After removing the supernatant solution, pure water was added to redisperse the PR122 microparticle dispersion liquid and again settled using a centrifugal separator. After performing the cleansing operation three times, the finally obtained PR122 microparticle dispersion liquid paste was vacuum dried at 25°C and -0.1 MPaG. Thus, PR122 microparticle dry powder was obtained. For the obtained PR122 microparticle dry powder, a TEM observation and an XRD measurement were performed to obtain the average particle diameter, the degree of crystallinity and the β-type crystal ratio. Further, for the respective values of the degree of crystallinity and the β-type crystal ratio, the ratio to the average particle diameter (Hereinafter referred to as degree of crystallinity/average particle diameter of the PR122 microparticle, and β-type crystal ratio/average particle diameter of the PR122 microparticle) was evaluated. This is to perform the standardization by being divided by the particle diameter because there is a difference in the scattering intensity in the XRD measurement due to the difference of the particle diameter of the PR122 microparticles in the XRD measurement. In the case of seeing the numerical change by being divided by the average particle diameter as described above in the present invention, it is preferable when the degree of variations of the particle diameter be within the range of 3 digit in the order of nanometers. More preferably, it stays within the range of 2 digit.

Table 1 shows the processing conditions (formulation and operating conditions) of the first fluid and the second fluid. Table 2 shows the preparation conditions of the second fluid and the obtained results. The target temperatures of the first fluid and the second fluid shown in Table 1 are preset temperatures of the temperature controller (heating and cooling) at the time of introducing the first fluid and the second fluid to the processing apparatus, respectively.

Table 1

| Item | | Setting |
|---|---|---|
| First fluid formulation | | MeOH |
| Second fluid formulation | | 3w% pigment/10wt% fuming sulfuric acid |
| Operation conditions | Number of rotations [rpm] | 2500 |
| | First fluid — Feed amount [ml/min] | 1000 |
| | First fluid — Temperature [°C] | 60 (Target temperature) |
| | Second fluid — Feed amount [ml/min] | 60 |
| | Second fluid — Temperature [°C] | 30 to 40 (Target temperature) |

[Table 2]

| Ex. | Second fluid preparation conditions | | | XRD measurement results | | Average particle diameter [nm] | Degree of crystallinity /average diameter | β-type crystal ratio/average particle diameter |
|---|---|---|---|---|---|---|---|---|
| | Preparation temp. [°C] | Preparation time [min] | Peripheral velocity [m/sec] | Degree of crystallinity [%] | β-type crystal ratio | | | |
| 1 | 26 | 30 | 18.85 | 51.13 | 62.65 | 36.4 | 1.40 | 1.72 |
| 2 | 45 | 30 | 18.85 | 38.77 | 49.54 | 21.3 | 1.82 | 2.33 |
| 3 | 60 | 30 | 18.85 | 54.91 | 34.03 | 12.3 | 4.46 | 2.77 |

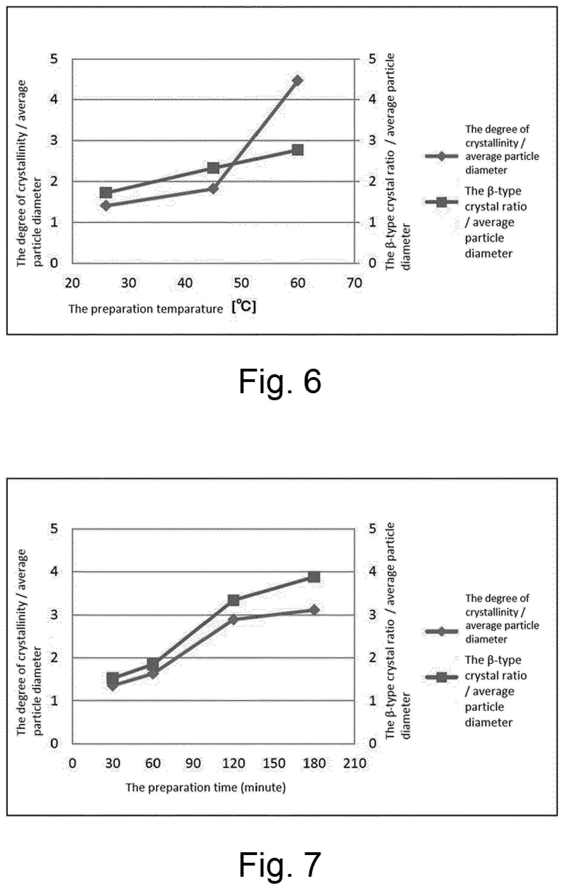

Fig. 6 shows the changes of the degree of crystallinity/average particle diameter and β-type crystal ratio/average particle diameter of the PR122 microparticle with respect to the preparation temperature of the second fluid in Examples 1 to 3. From Fig. 6, it is recognized that there is a tendency that when the preparation temperature of the second fluid increases, all of the numerical values of the degree of crystallinity/average particle diameter, β-type crystal ratio/average particle diameter of the PR122 microparticles with respect to the preparation temperature of the second fluid in Examples 1 to 3 increase.

Further, from Table 2, it is recognized that when the preparation temperature of the second fluid increases, the average particle diameter of the obtained PR122 microparticles decreases.

(Examples 4 to 16)

Dry powder of PR122 microparticles was obtained in the same manner as in Examples 1 to 3 except that the preparation conditions of the pigment solution were set to any of the conditions shown in Tables 3 to 5. The results are shown in Tables 3 to 5. In Examples 4 to 16, the agitation energy was increased and decreased by changing the agitation time (preparation time) of the pigment solution as the second fluid.

[Table 3]

| Ex. | Second fluid preparation conditions | | | XRD measurement results | | Average particle diameter [nm] | Degree of crystallinity / Average particle diameter | β-type crystal ratio / Average particle diameter |
|---|---|---|---|---|---|---|---|---|
| | Preparation temp. [°C] | Preparation time [min] | Peripheral velocity [m/sec] | Degree of crystallinity [%] | β-type crystal ratio [%] | | | |
| 4 | 28 | 30 | 18.85 | 55.73 | 62.66 | 41.2 | 1.35 | 1.52 |
| 5 | 28 | 60 | 18.85 | 55.54 | 62.81 | 34.1 | 1.63 | 1.84 |
| 6 | 28 | 120 | 18.85 | 53.15 | 61.37 | 18.4 | 2.89 | 3.34 |
| 7 | 28 | 180 | 18.85 | 48.47 | 60.51 | 15.6 | 3.11 | 3.88 |

[Table 4]

| Ex. | Second fluid preparation conditions | | | XRD measurement results | | Average particle diameter [nm] | Degree of crystallinity / Average particle diameter | β-type crystal ratio / Average particle diameter |
|---|---|---|---|---|---|---|---|---|
| | Preparation temp. [°C] | Preparation time [min] | Peripheral velocity [m/sec] | Degree of crystallinity [%] | β-type crystal ratio [%] | | | |
| 8 | 32 | 30 | 18.85 | 50.74 | 63.73 | 41.2 | 1.23 | 1.55 |
| 9 | 33 | 60 | 18.85 | 53.49 | 62.72 | 38.5 | 1.39 | 1.63 |
| 10 | 32 | 120 | 18.85 | 48.87 | 63.12 | 18.5 | 2.64 | 3.41 |
| 11 | 32 | 180 | 18.85 | 38.72 | 41.35 | 11.1 | 3.49 | 3.73 |

[Table 5]

| Ex. | Second fluid preparation conditions | | | XRD measurement results | | Average particle diameter [nm] | Degree of crystallinity / Average particle diameter | β-type crystal ratio / Average particle diameter |
|---|---|---|---|---|---|---|---|---|
| | Preparation temp. [°C] | Preparation time [min] | Peripheral velocity [m/sec] | Degree of crystallinity [%] | β-type crystal ratio | | | |
| 12 | 28 | 30 | 25.13 | 61.23 | 64.61 | 39.4 | 1.55 | 1.64 |
| 13 | 28 | 60 | 25.13 | 54.52 | 66.55 | 31.2 | 1.75 | 2.13 |
| 14 | 28 | 90 | 25.13 | 56.72 | 64.96 | 28.4 | 2.00 | 2.29 |
| 15 | 28 | 120 | 25.13 | 50.71 | 60.86 | 20.2 | 2.51 | 3.01 |
| 16 | 28 | 180 | 25.13 | 38.74 | 65.41 | 14.15 | 2.74 | 4.62 |

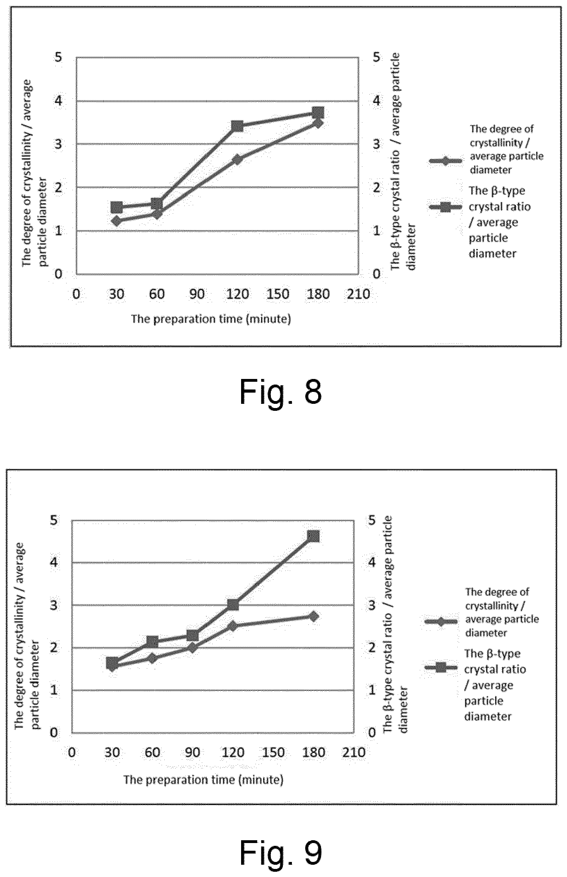

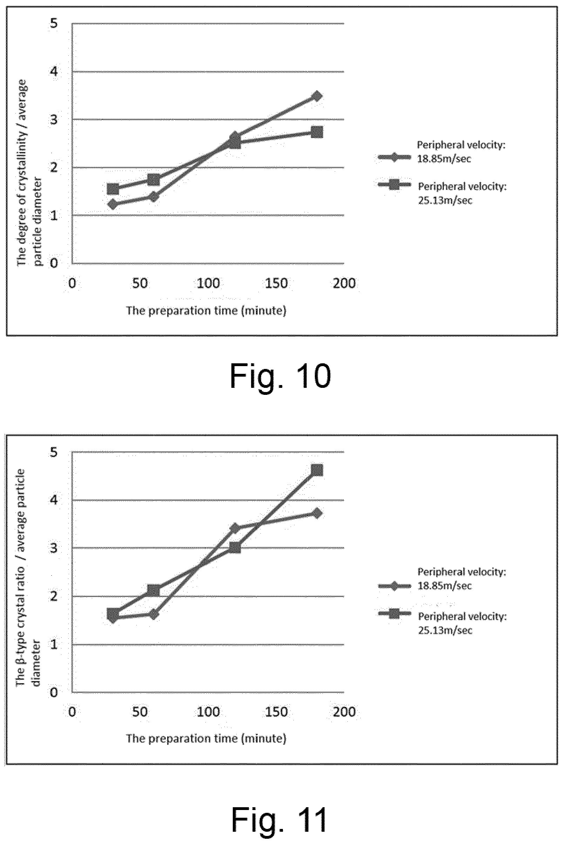

For Examples 4 to 7, the changes of the degree of crystallinity/average particle diameter and β-type crystal ratio/average particle diameter of the PR122 microparticles with respect to the preparation time of the second fluid is shown in Fig. 7. For Examples 8 to 11, the changes of the degree of crystallinity/average particle diameter and β-type crystal ratio/average particle diameter of the PR122 microparticles with respect to the preparation time of the second fluid is shown in Fig. 8. For Examples 12 to 16, the changes of the degree of crystallinity/average particle diameter and the β-type crystal ratio/average particle diameter of the PR122 microparticles with respect to the preparation time of the second fluid is shown in Fig. 9. Further, for Examples 8 to 11 and Examples 12, 13, 15 and 16, the changes of the degree of crystallinity/average particle diameter of the PR122 microparticles with respect to the preparation time of the second fluid are shown in Fig. 10. For Examples 8 to 11 and Examples 12, 13, 15 and 16, the changes of the β-type crystal ratio/average particle diameter of the PR122 microparticles with respect to the preparation time of the second fluid are shown in Fig. 11.

From Figs. 7 to 9, it is recognized that when the preparation time of the second fluid increases, both the numerals of the degree of crystallinity/average particle diameter and the β-type crystal ratio/average particle diameter of the obtained PR122 microparticles increase. Further, from Tables 3 to 5, it is recognized that when the preparation temperature of the second fluid increases, the average particle diameter of the obtained PR122 microparticles decreases.

From Figs. 10 to 11, it is recognized that even in the examples in which the peripheral velocity of the agitating blade was changed at the time of preparing the pigment solution as the second fluid in addition to the preparation time of the second fluid, when the preparation time of the second fluid increases, both the numerals of the degree of crystallinity/average particle diameter and the β-type crystal ratio/average particle diameter of the obtained PR122 microparticles increase.

(Examples 17 to 22)

Dry powder of PR122 microparticles was obtained in the same manner as in Examples 1 to 3 except that the preparation conditions of the pigment solution were set to the conditions shown in Tables 6 or 7. The results are shown in Tables 6 to 7. In Examples 17 to 22, the agitation energy was increased and decreased by changing the peripheral velocity of the agitating blade at the time of preparing the pigment solution as the second fluid.

[Table 6]

| Ex. | Second fluid preparation conditions | | | XRD measurement results | | Average particle diameter [nm] | Degree of crystallinity / Average particle diameter | β-type crystal ratio / Average particle diameter |
|---|---|---|---|---|---|---|---|---|
| | Preparation temp. [°C] | Preparation time [min] | Peripheral velocity [m/sec] | Degree of crystallinity [%] | β-type crystal ratio [%] | | | |
| 17 | 28 | 30 | 18.85 | 55.73 | 62.66 | 41.2 | 1.35 | 1.52 |
| 18 | 28 | 30 | 25.13 | 61.23 | 64.61 | 39.4 | 1.55 | 1.64 |
| 19 | 28 | 30 | 31.42 | 63.08 | 64.17 | 24.6 | 2.56 | 2.61 |

[Table 7]

| Ex. | Second fluid preparation conditions | | | XRD measurement results | | Average particle diameter [nm] | Degree of crystallinity / Average particle diameter | β-type crystal ratio / Average particle diameter |
|---|---|---|---|---|---|---|---|---|
| | Preparation temp. [°C] | Preparation time [min] | Peripheral velocity [m/sec] | Degree of crystallinity [%] | β-type crystal ratio [%] | | | |
| 20 | 28 | 60 | 18.85 | 55.54 | 62.18 | 34.1 | 1.63 | 1.84 |
| 21 | 28 | 60 | 25.13 | 54.52 | 66.55 | 31.2 | 1.75 | 2.13 |
| 22 | 28 | 60 | 31.42 | 60.75 | 62.58 | 21.3 | 2.85 | 2.94 |

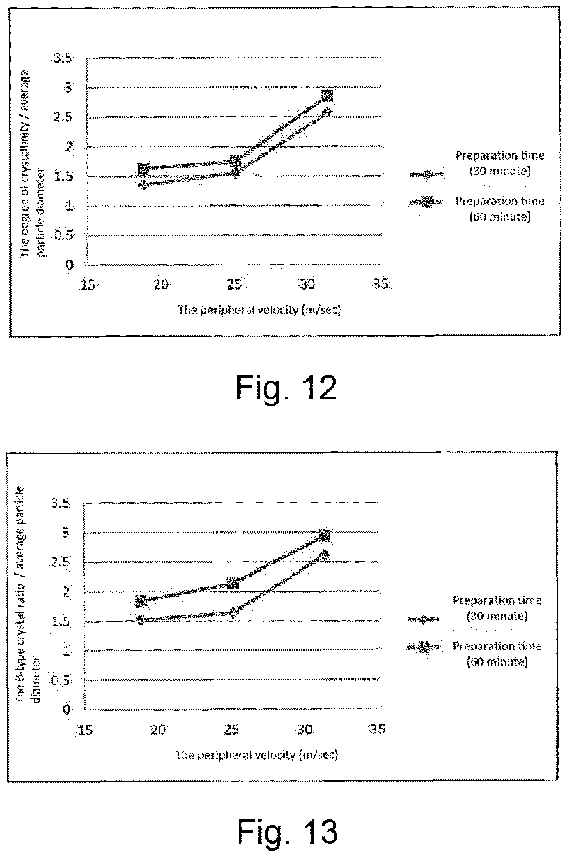

For Examples 17 to 22, the changes of the degree of crystallinity/average particle diameter of the PR122 microparticles with respect to the peripheral velocity of the agitating blade at the time of preparing the second fluid are shown in Fig. 12. For Examples 17 to 22, the changes of the β-type crystal ratio/average particle diameter of the PR122 microparticles with respect to the peripheral velocity of the agitating blade at the time of preparing the second fluid are shown in Fig. 13.

From FIGS. 12 to 13, it is recognized that when the peripheral velocity of the agitating blade at the time of preparing the second fluid increases, both the numerals of the degree of crystallinity/average particle diameter and the β-type crystal ratio/average particle diameter of the obtained PR122 microparticles increase, and, it was also recognized that there was the same tendency even in the Examples in which the preparation time of the second fluid was changed in addition to the changes of the peripheral velocity.

Further, from Tables 6 to 7, it is recognized that when the peripheral velocity of the agitating blade at the time of preparing the second fluid increases, the average particle diameter of the obtained PR122 microparticles decreases.

In the present invention, from the results of the above-mentioned Examples, to increase and decrease the agitation energy, the following can be derived as to the setting of the priority of the preparation condition of the second fluid.

From the results of Examples 1 to 3, when it was carried out while maintaining the preparation temperature of the second fluid at 60°C (Example 3), the agitation time for the preparation was as short as 30 minutes, and the peripheral velocity of the agitating blade of the agitator was a relative slow velocity of 18.85 m/sec. However, comparing to the other cases (Examples 13 to 16) in which a agitation time longer than the above case was spend and the temperature at the time of the preparation when the peripheral velocity was held more higher was relatively low, in Example 3, microparticles having smaller in average particle diameter with respect to the obtained PR122 could be obtained, and the influence that the difference of the preparation temperature impacts on the prioperties/characteristics of microparticles was the strongest. In Example 16 in which the agitation time was long enough 180 min, the results became the same as in Example 3, and microparticles small in average particle diameter with respect to the obtained PR122 could be obtained. Further, from the comparison of Example 3 and Examples 21 and 22, in the case where the preparation temperature of the second fluid is relatively low, even if the peripheral velocity is maintained much higher and a much longer agitation time is spent, the average particle diameter of the obtained PR122 cannot be smaller as in Example 3. In other words, only if the preparation temperature of the second fluid is maintained higher like Example 3, even if the agitation time is shortened and the peripheral velocity is kept low, microparticles small in average particle diameter can be obtained.

From the results of Examples 4 to 16, even if the preparation temperature of the second fluid is maintained relatively low and the peripheral velocity of the agitating blade of the agitator is set to be relatively low, by securing a sufficiently longer agitation time (e.g., Example 7 or Example 11), the result becomes the same as in Example 3, and microparticles small in average particle diameter regarding the obtained PR122 can be obtained. Therefore, following the preparation temperature, the length of the agitation time has more stronger influence to the properties/characteristics of microparticles. In other words, like Example 7 and Example 11, even if the preparation temperature of the second fluid is relatively low and the peripheral velocity is relatively low, by a securing significantly long agitation time, microparticles small in average particle diameter which is equivalent to the average particle diameter of the PR122 microparticles obtained in Example 3 can be obtained. Further, like Examples 17 to 22, comparing the relatively shorter agitation times, if the peripheral velocity of the agitating blade of the agitator is same, microparticles small in average particle diameter regarding the obtained PR122 can be obtained. Therefore, the influence that the length of the agitation time exerts the properties/characteristics of microparticles is apparent.

From the results of Examples 17 to 22, the changes of the peripheral velocity of the agitating blade of the agitator has not so strong influence exerting to the average particle diameter of the obtained PR122 microparticles. Comparing Example 17 and Example 18, and Example 20 and Example 21, which are the same in agitation time, when the peripheral velocity is 18.85 m/sec and 25.13 m/sec, there is no big difference in the average particle diameter of the obtained PR122 microparticles. In Example 19 and Example 22 in which the peripheral velocity is 31.42 m/sec, there exists a difference in average particle diameter of the obtained PR122 microparticles as compared to the above-mentioned Examples in which the peripheral velocity is 18.85 m/sec and 25.13 m/sec. However, the average particle diameter of the obtained PR122 microparticles does not affect the results of Examples 1 to 3 and Examples 4 to 16.

Further, as to the relationship of the above description and the degree of crystallinity and the β-type crystal ratio, the correspondence relationship becomes the same. Because when the degree of crystallinity and the β-type crystal ratio is not divided by the average particle diameter, there appeared no clear numerical change, but a clear numerical change became apparent for the first time by seeing the degree of crystallinity/average particle diameter, β-type crystal ratio/average particle diameter. Even if the change amount of the degree of crystallinity and the β-type crystal ratio is small, since the change amount of the average particle diameter is large, a difference occurs as a result when divided. It is judged that the larger the numerical value of the degree of crystallinity/average particle diameter and the β-type crystal ratio/average particle diameter is, the better it is. When the particle becomes small like microparticles, as the XRD measurement result, the peak tends to become broad, which in turn tends to cause a lower degree of crystallinity and β-type crystal ratio. For this reason, when evaluating the degree of crystallinity and the β-type crystal ratio, it is required to consider the relationship to the particle diameter.

The "degree of crystallinity" and the "β-type crystal ratio" which becomes an index of evaluating the the particle diameter, color hue, coloring power, durability of the targeted microparticles naturally differ depending on the use of the microparticles, and therefore it is enough to perform a control depending on the application of the microparticles.

Further, in the case of seeing the numerical change by being divided by the average particle diameter as described above in the present invention, it is preferable when the degree of variations of the particle diameter be within the range of 3 digit in the order of nanometers. More preferably, it stays within the range of 2 digit.

(Production of PR122 microparticle using an alkaline pasting method)(Examples 23 to 40)

Using a fluid processing apparatus shown in Fig. 1, a pigment solution as a microparticle raw material solution and a separating solution were mixed in a thin film fluid formed between processing surfaces 1 and 2, which are arranged so as to face each other and provided with processing surfaces capable of approaching and separating with each other, at least one of the processing surfaces being capable of rotating with respect to the other. Thus, pigment microparticles were separated in the thin film fluid. The preparation of the pigment solution was performed using a rotating agitator having agitating blades, and the agitation energy was increased and decreased by changing the temperature of the pigment solution (preparation temperature) and/or the agitation time(preparation time) among three conditions (agitation time, peripheral velocity of the agitating blade, temperature of microparticle raw material solution) deciding the agitation energy.

Initially, using the agitator (CLEARMIX (manufactured by M Technique Co., Ltd.)) having rorating agitating blades shown in FIG. 4 and FIG. 5, a pigment solution was prepared. Concretely, using CLEARMIX, PR122 was introduced in a mixed solvent mixed at the weight ratio of dimethyl sulfoxide/pure water (PW)/potassium hydroxide (KOH)=73.5/23.17/1.37 while agitating the solvent, and the pigment solution was agitated for the preparation time and at the preparation temperature shown in Table 9 or Table 10 so that the ratio by weight became dimethyl sulfoxide/pure water (PW)/potassium hydroxide (KOH) /PR122=73.5/23.17/1.37/1.96 (1.96 wt% PR122 solution) to thereby prepare a 1.96 wt% PR122 solution.

Next, while supplying 20 wt% acetic acid/methanol (MeOH) as a separating solution of the first fluid from the center at the supply pressure/back pressure = 0.121 MPaG/0.020 MPaGm and the number of rotations of 2500 rpm, the pigment solution was introduced into between the processing surfaces as a second fluid, and the first fluid and the second fluid was mixed in the thin film fluid. PR122 microparticle dispersion liquid was discharged from between the processing surfaces 1 and 2. In order to remove the impurities from the discharged PR122 microparticle dispersion liquid, the PR122 microparticle dispersion liquid was softly agglutinated, and the PR122 microparticle dispersion liquid was settled by a centrifugal separator (x18000G) as a cleansing operation. After removing the supernatant solution, pure water was added to redisperse the PR122 microparticle dispersion liquid and again settled using a centrifugal separator. After performing the cleansing operation three times, the finally obtained PR122 microparticle dispersion liquid paste was vacuum dried at 50°C and -0.1 MPaG. Thus, PR122 microparticle dry powder was obtained. For the obtained PR122 microparticle dry powder, a TEM observation and an XRD measurement were performed to obtain the average particle diameter, the degree of crystallinity and the β-type crystal ratio. Further, for the respective values of the degree of crystallinity and the β-type crystal ratio, in the same manner as in Examples 1 to 22, the ratio to the average particle diameter (hereinafter referred to as degree of crystallinity/average particle diameter of the PR122 microparticle, and β-type crystal ratio/average particle diameter of the PR122 microparticle) was evaluated.

Tables 8 to 10 shows the processing conditions(formulation and operating condition) of the first fluid and the second fluid, the preparation condition of the second fluid, and the obtained results.

The temperatures of the first fluid and the second fluid shown in Table 8 are preset temperatures of the temperature controller (heating and cooling) at the time of introducing the first fluid and the second fluid to the processing apparatus, respectively, and the temperatures of the first fluid and the second fluid shown in Tables 9 or 10 is a temperature measured immediately before introducing (in more detail, immediately before introducing into between the processing surfaces), the first fluid and the second fluid to the processing apparatus, respectively.

[Table 8]

| Item | | | Setting (1) | Setting (2) |
|---|---|---|---|---|
| First fluid formulation | | | 20 wt% acetic acid / MeOH | 20 wt% acetic acid / MeOH |
| Second fluid formulation | | | 1.96 wt% pigment/73.5 wt% DMSO/23.17 wt% PW/1.37 wt% KOH | 1.96 wt% pigment/73.5 wt% DMSO/23.17 wt% PW/1.37 wt% KOH |
| Operation condition | | Number of rotation [rpm] | 2,500 | 2,500 |
| | First fluid | Feeding amount [ml/min] | 1,000 | 1,000 |
| | | Temperature [°C] | 30 (target temperature) | 60 (target temperature) |
| | Second fluid | Feeding amount [ml/min] | 60 | 60 |
| | | Temperature [°C] | 30 (target temperature) | 30 (target temperature) |

[Table 9]

| Ex. | Second fluid preparation condition | | | First fluid feeding condition | | Second fluid feeding condition | | XRD | | Average particle diameter [nm] | degree of crystallinity/average particle diameter | β-type crystal ratio/average particle diameter |
|---|---|---|---|---|---|---|---|---|---|---|---|---|
| | Preparation temperature [°C] | Preparation time [min] | Peripheral velocity [m/min] | Setting [ml/min] | Temperature [°C] | Setting [ml/min] | Temperature [°C] | Degree of crystallinity [%] | β-type crystal ratio [%] | | | |
| 23 | 60 | 30 | 31.42 | 1,000 | 28.6 | 60 | 28.6 | 67.69 | 77.98 | 26.7 | 2.54 | 2.92 |
| 24 | 60 | 60 | 31.42 | 1,000 | 28.4 | 60 | 28.4 | 71.15 | 77.46 | 18.6 | 3.83 | 4.16 |
| 25 | 60 | 90 | 31.42 | 1,000 | 28.4 | 60 | 28.4 | 70.52 | 75.33 | 17.4 | 4.05 | 4.33 |
| 26 | 45 | 30 | 31.42 | 1,000 | 28.6 | 60 | 28.4 | 73.68 | 77.12 | 25.4 | 2.90 | 3.04 |
| 27 | 45 | 60 | 31.42 | 1,000 | 28.4 | 60 | 28.6 | 79.18 | 74.05 | 21.3 | 3.72 | 3.48 |
| 28 | 45 | 90 | 31.42 | 1,000 | 28.3 | 60 | 28.3 | 51.67 | 74.47 | 15.4 | 3.36 | 4.84 |
| 29 | 28 | 30 | 31.42 | 1,000 | 27.1 | 60 | 28.4 | 60.01 | 76.65 | 32.1 | 1.87 | 2.39 |
| 30 | 28 | 60 | 31.42 | 1,000 | 26.1 | 60 | 28.1 | 67.11 | 76.49 | 24.6 | 2.73 | 3.11 |
| 31 | 28 | 90 | 31.42 | 1,000 | 27.4 | 60 | 28.9 | 65.48 | 76.34 | 15.4 | 4.25 | 4.96 |

[Table 10]

| Ex. | Second fluid preparation condition | | | First fluid feeding condition | | Second fluid feeding condition | | XRD | | Average particle diameter [nm] | degree of crystallinity/average particle diameter | β-type crystal ratio/average particle diameter |
|---|---|---|---|---|---|---|---|---|---|---|---|---|
| | Preparation temperature [°C] | Preparation time [min] | Peripheral velocity [m/min] | Setting [ml/min] | Temperature [°C] | Setting [ml/min] | Temperature [°C] | Degree of crystallinity [%] | β-type crystal ratio [%] | | | |
| 32 | 60 | 30 | 31.42 | 1,000 | 61.2 | 60 | 28.3 | 75.79 | 78.01 | 28.9 | 2.62 | 2.70 |
| 33 | 60 | 60 | 31.42 | 1,000 | 61.2 | 60 | 28.4 | 65.26 | 78.16 | 21.3 | 3.06 | 3.67 |
| 34 | 60 | 90 | 31.42 | 1,000 | 61.9 | 60 | 28.6 | 66.06 | 76.17 | 16.4 | 4.03 | 4.64 |
| 35 | 45 | 30 | 31.42 | 1,000 | 61.2 | 60 | 28.3 | 64.85 | 76.75 | 26.4 | 2.46 | 2.91 |
| 36 | 45 | 60 | 31.42 | 1,000 | 60.3 | 60 | 28.3 | 64.98 | 75.2 | 16.4 | 3.96 | 4.59 |
| 37 | 45 | 90 | 31.42 | 1,000 | 61.2 | 60 | 28.6 | 59.5 | 74.41 | 11.2 | 5.31 | 6.64 |
| 33 | 28 | 30 | 31.42 | 1,000 | 61.1 | 60 | 28.2 | 51.69 | 74.41 | 27.4 | 1.89 | 2.72 |
| 39 | 28 | 60 | 31.42 | 1,000 | 62.3 | 60 | 28.6 | 59.5 | 74.41 | 21.3 | 2.79 | 3.49 |
| 40 | 28 | 90 | 31.42 | 1,000 | 63.1 | 60 | 29.1 | 61.23 | 74.41 | 11.2 | 5.47 | 6.64 |

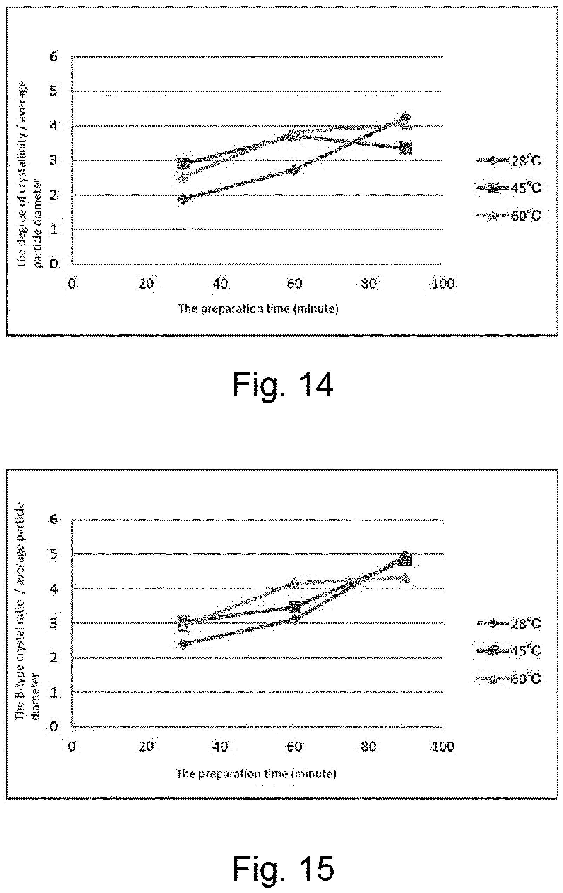

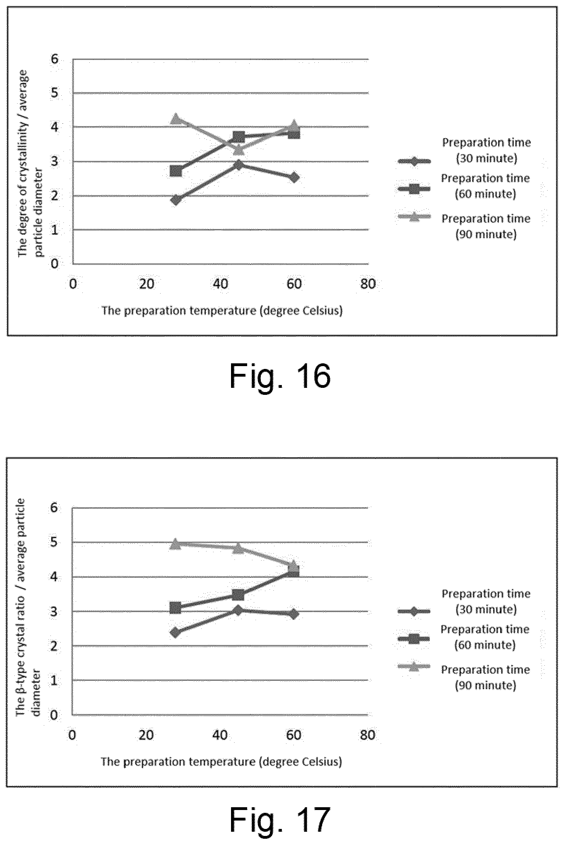

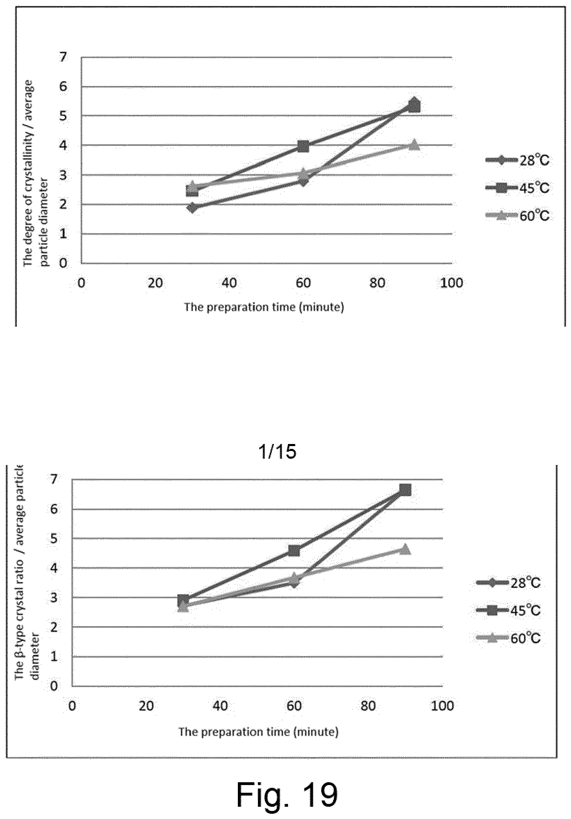

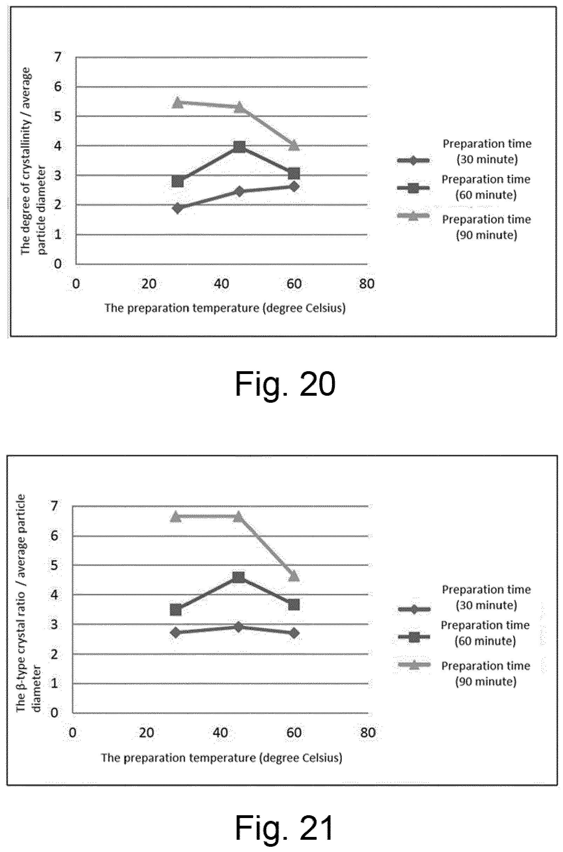

For Examples 23 to 31, the changes of the degree of crystallinity/average particle diameter of the PR122 microparticles with respect to the preparation time of the second fluid is shown in Fig. 14. For Examples 23 to 31, the changes of the β-type crystal ratio/average particle diameter of the PR122 microparticles with respect to the preparation time of the second fluid is shown in Fig. 15. For Examples 23 to 31, the changes of the degree of crystallinity/average particle diameter of the PR122 microparticles with respect to the preparation tempareture of the second fluid is shown in Fig. 16. For Examples 23 to 31, the changes of the β-type crystal ratio/average particle diameter of the PR122 microparticles with respect to the preparation temperature of the second fluid is shown in Fig. 17. Further, for Examples 32 to 40, the changes of the degree of crystallinity/average particle diameter of the PR122 microparticles with respect to the preparation time of the second fluid is shown in Fig. 18. For Examples 32 to 40, the changes of the β-type crystal ratio/average particle diameter of the PR122 microparticles with respect to the preparation time of the second fluid is shown in Fig. 19. For Examples 32 to 40, the changes of the degree of crystallinity/average particle diameter of the PR122 microparticles with respect to the preparation temperature of the second fluid is shown in Fig. 20. For Examples 32 to 40, the changes of the β-type crystal ratio/average particle diameter of the PR122 microparticles with respect to the preparation temperature of the second fluid is shown in Fig. 21.

From FIGS. 14 to 15, and FIG. 18 to FIG. 19, it is recognized that when the preparation time of the second fluid increases, there is a tendency that both the numerals of the degree of crystallinity/average particle diameter and the β-type crystal ratio/average particle diameter of the obtained PR122 microparticles increase, and it was also recognized that there was the same tendency even in the Examples in which the preparation temperature of the second fluid was changed in addition to the changes of the preparation time.

From Figs. 16 to FIG. 17, and FIG. 20 to FIG. 21, it is recognized that when the preparation temperature the second fluid increases, both the numerals of the degree of crystallinity/average particle diameter and the β-type crystal ratio/average particle diameter of the obtained PR122 microparticles cange. Even in Examples in which the preparation temperature of the second fluid was changed in addition to the change of the preparation time, the same tendency was recognized.

Further, from Tables 9 to 10, it is recognized that when the preparation time of the second fluid increases, the average particle diameter of the obtained PR122 microparticles decreases. Further, from Table 9, it is recognized that when the preparation temperature of the second fluid increases, there is a tendency that the average particle diameter of the obtained PR122 microparticles decreases.

In Examples of this application, as to the obtained PR122 microparticle, the β-type crystal ratio was obtained, and then the β-type crystal ratio/average particle diameter was evaluated. However, it may be configured such that the component ratio (α-type crystal ratio) of a particular crystal form which is an α-type crystal is obtained, and then the α-typecrystal ratio/average particle diameter is evaluated. In Examples of this application, there is a tendency that the variation of the average particle diameter becomes larger than the variation of the β-type crystal ratio or the α-type crystal ratio which changes depending on the increase/decrease of the agitation energy by changing at least one of three conditions (the agitation time, the peripheral velocity of the agitating blade, the temperature of the microparticle raw material solution) deciding the agitation energy because there is the similar tendency even in the case of evaluating either of the β-type crystal ratio/average particle diameter and the α-typecrystal ratio/average particle diameter.

One example of the production method of the present invention may be carried out as follows. In producing microparticles in which the particle diameter, the degree of crystallinity, and the crystal form are set to specific conditions, the peripheral velocity condition of the agitating blade in the dissolving step is changed. With this, the peripheral velocity satisfying the specific conditions on the particle diameter of the microparticles in the separating step is decided. While maintaining the decided peripheral velocity conditions, at least either one of the agitation time condition and the temperature condition is changed to determine the agitation time condition and the temperature condition satisfying the specific conditions on the decree of crystallinity and the crystal form of the microparticles in the separating step. With this, microparticles in which the particle diameter, the degree of crystallinity, and the crystal form satisfy the specific conditions can be produced.

In Examples 17 to 22, by changing the peripheral velocity of the agitating blade at the time of preparing the pigment solution as the second fluid, the peripheral velocity condition satisfying the specific conditions on the average particle diameter of the obtained PR122 microparticles is determined. Here, the specific condition is considered as Example 17 which is the largest in average particle diameter of PR122 microparticls, and 18.85 m/sec which is the peripheral velocity is determined as the peripheral velocity condition.

Next, in Examples 4 to 11 of this application, by changing at least either one of the preparation time(agitation time) and the preparation temperature of the second fluid while maintaining the peripheral velocity condition, the agitation time condition and the temperature condition satisfying the specific conditions on the degree of crystallinity and the crystal form of the PR122 microparticle is determined. Here, Example 4 (Example 17) in which the "degree of crystallinity" is high and the "β-type crystal ratio" is high is considered as the specific condition, and 30 minutes which is the preparation time (agitation time) is determined as the agitation time condition.

Further, in Examples 1 to 3 and 4 (Example 17), Example 4 (Example 17) in which the "degree of crystallinity" is high and the "β-type crystal ratio" is high is considered as the specific conditions, and 28°C which is the temperature (preparation temperature) is determined as the temperature condition.

Then, it can be carried out to produce microparticles in which the particle diameter, the degree of crystallinity, and the crystal form satisfy the specific conditions.

The settings of the specific conditions are merely one example, and is not limited to the above-mentioned one example.

(Production of indomethacin microparticles using a poor solvent method) (Examples 41 to 49)

Using a fluid processing apparatus shown in Fig. 1, an indomethacin solution as a microparticle raw material solution and a separating solution were mixed in a thin film fluid formed between processing surfaces 1 and 2, which are arranged so as to face each other and provided with processing surfaces capable of approaching to and separating from with each other, at least one of the processing surfaces being capable of rotating with respect to the other. Thus, indomethacin microparticles were separated in the thin film fluid. The preparation of the indomethacin solution was performed using a agitator having rotating agitating blades, and the agitation energy was increased and decreased by changing the temperature (preparation temperature) of the indomethacin solution and/or the agitation time(preparation time) among three conditions (agitation time, peripheral velocity of the agitating blades, temperature of the microparticle raw material solution) determining the agitation energy.

Initially, using the agitator (CLEARMIX (manufactured by M Technique Co., Ltd.)) having rotating agitating blades shown in FIG. 4 and FIG. 5, an indomethacin solution was prepared. Concretely, using CLEARMIX, the indomethacin was introduced while agitating diethyl ether at the peripheral velocity of the agitating blade shown in Table 12, and the indomethacin solution was agitated for the preparation time at the preparation temperature shown in Table 12. Thus, 1.5 wt% indomethacin solution was prepared.

Next, while supplying hexane as a separating solution of the first fluid from the center at the supply pressure/back pressure = 0.089 MPaG/0.020 MPaG, and the number of rotations of 1,700 rpm, the indomethacin solution was introduced into between the processing surfaces as a second fluid, and the first fluid and the second fluid were mixed in the thin film fluid. The indomethacin microparticle dispersion liquid was made to discharge from between the processing surfaces 1 and 2. In order to remove the impurities from the discharged indomethacin microparticle dispersion liquid, the indomethacin microparticle dispersion liquid was softly agglutinated, and the indomethacin microparticle dispersion liquid was settled by a centrifugal separator (x8000G) as a cleansing operation. After removing the supernatant solution, pure water was added to redisperse the indomethacin microparticle dispersion liquid and again settled using a centrifugal separator. After performing the cleansing operation three times, the finally obtained indomethacin microparticle dispersion liquid paste was vacuum dried at 25°C and -0.1 MPaG. Thus, indomethacin microparticle dry powder was obtained. For the obtained indomethacin microparticle dry powder, a TEM observation and an XRD measurement were performed to obtain the average particle diameter, the degree of crystallinity and the $\gamma$-type crystal ratio. Further, for the respective values of the degree of crystallinity and the $\gamma$-type crystal ratio, in the same manner as in Examples 1 to 40, the ratio to the average particle diameter (hereinafter referred to as the degree of crystallinity/average particle diameter of the indomethacin microparticle, and $\gamma$-type crystal ratio/average particle diameter of the indomethacin microparticle) was evaluated.

Tables 11 to 12 show the processing conditions(formulation and operating conditions) of the first fluid and the second fluid, the preparation conditions of the second fluid, and the obtained results.

The temperatures (target temperature) of the first fluid and the second fluid shown in Table 11 are preset temperatures of the temperature controller (heating and cooling) at the time of introducing the first fluid and the second fluid to the processing apparatus, respectively, and the temperatures of the first fluid and the second fluid shown in Table 12 are temperatures measured immediately before introducing (in more detail, immediately before introducing into between the processing surfaces) the first fluid and the second fluid to the processing apparatus, respectively.

[Table 11]

| Item | | | Setting |
|---|---|---|---|
| First fluid formulation | | | Hexane |
| Second fluid formulation | | | 1.5 wt% indomethacin/ diethyl ether |
| Operation condition | Number of rotation [rpm] | | 1,700 |
| | First fluid | Feeding amount [ml/min] | 400 |
| | | Temperature [°C] | 60 |
| | Second fluid | Feeding amount [ml/min] | 10 |
| | | Temperature [°C] | 25 |

[Table 12]

| Ex. | Second fluid preparation condition | | | First fluid feeding condition | | Second fluid feeding condition | | XRD | | Average particle diameter [nm] | degree of crystallinity/average particle diameter | γ-type crystal ratio/average particle diameter |
|---|---|---|---|---|---|---|---|---|---|---|---|---|
| | Preparation temperature [°C] | Preparation time [min] | Peripheral velocity [m/sec] | Setting [ml/min] | Temperature [°C] | Setting [ml/min] | Temperature [°C] | Degree of crystallinity [%] | γ-type crystal ratio [%] | | | |
| 41 | 33 | 15 | 31.42 | 400 | 60.1 | 10 | 25.1 | 66.45 | 39.12 | 425.3 | 0.156 | 0.092 |
| 42 | 33 | 30 | 31.42 | 400 | 60.4 | 10 | 25.3 | 68.45 | 40.12 | 346.9 | 0.197 | 0.116 |
| 43 | 33 | 60 | 31.42 | 400 | 60.2 | 10 | 25.4 | 67.45 | 40.06 | 276.4 | 0.244 | 0.145 |
| 44 | 25 | 15 | 31.42 | 400 | 60.1 | 10 | 25.6 | 71.12 | 33.26 | 354.6 | 0.201 | 0.094 |
| 45 | 25 | 30 | 31.42 | 400 | 60.2 | 10 | 25.1 | 78.12 | 33.15 | 246.5 | 0.317 | 0.134 |
| 46 | 25 | 60 | 31.42 | 400 | 60.5 | 10 | 24.9 | 68.1 | 32.96 | 165.4 | 0.412 | 0.199 |
| 47 | 5 | 15 | 31.42 | 400 | 60.3 | 10 | 24.6 | 62.3 | 30.06 | 245.6 | 0.254 | 0.122 |
| 48 | 5 | 30 | 31.42 | 400 | 60.1 | 10 | 24.8 | 68.7 | 30.09 | 215.6 | 0.319 | 0.140 |
| 49 | 5 | 60 | 31.42 | 400 | 60.1 | 10 | 25.3 | 64.5 | 30.12 | 114.6 | 0.563 | 0.263 |

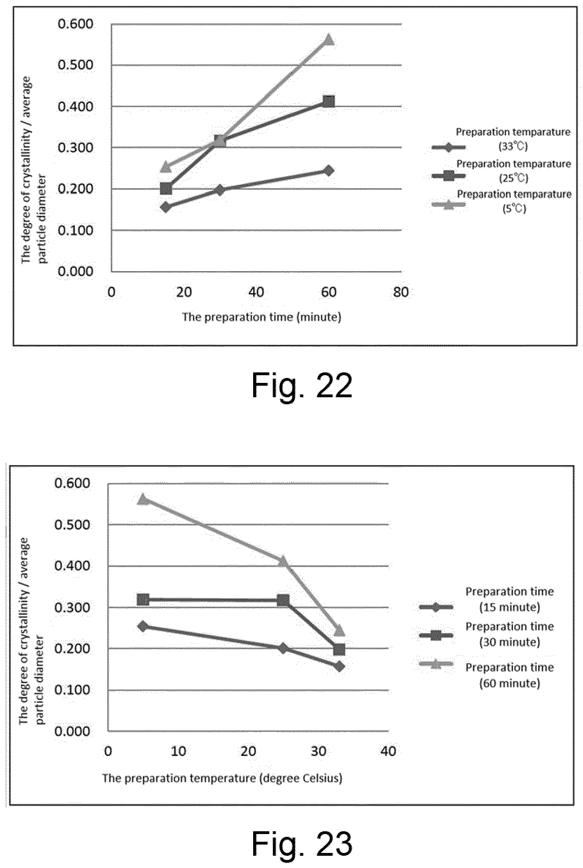

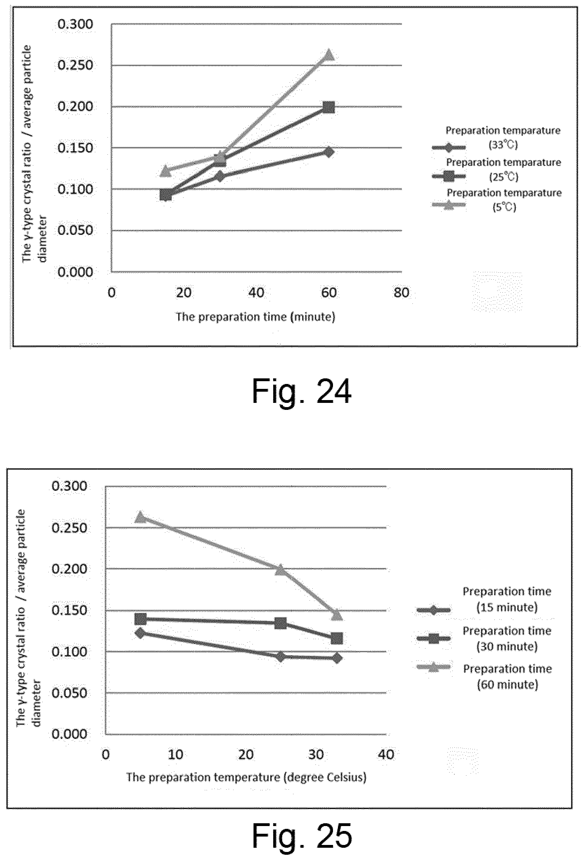

For Examples 41 to 49, the changes of the degree of crystallinity/average particle diameter of the indomethacin microparticles with respect to the preparation time of the second fluid is shown in Fig. 22. For Examples 41 to 49, the changes of the degree of crystallinity/average particle diameter of the indomethacin microparticles with respect to the preparation temperature of the second fluid is shown in Fig. 23. Further, for Examples 41 to 49, the changes of the γ-type crystal ratio/average particle diameter of the indomethacin microparticles with respect to the preparation time of the second fluid are shown in Fig. 24. For Examples 41 to 49, the changes of the γ-type crystal ratio/average particle diameter of the indomethacin microparticles with respect to the preparation temperature of the second fluid is shown in Fig. 25.

For the preparation condition of the second fluid, when comparing the same preparation temperature (Examples 41 to 43, Examples 44 to 46, and Examples 47 to 49), for any of the temperature of 33°C, 25°C, and 5°C, there were tendencies that the average particle diameter of the obtained indomethacin microparticles decreased and the degree of crystallinity/average particle diameter and the γ-type crystal ratio/average particle diameter increases as the preparation time increased.

Further, for the preparation condition of the second fluid, when comparing the same preparation time (Examples 41, 44, and 47, Examples 42, 45, 48 and 43, 46, and 49), for any of 15 minutes, 30 minutes, and 60 minutes, there were tendencies that the average particle diameter of the obtained indomethacin microparticles increased and both the degree of crystallinity/average particle diameter and the γ-type crystal ratio/average particle diameter decreased as the preparation temperature increased.

With reference to FIG. 22 and FIG. 24, while the preparation time of the second fluid is short (when the preparation time is 15 minutes), it can be understood that there are almost no difference in the degree of crystallinity/average particle diameter and the γ-type crystal ratio/average particle diameter of the obtained indomethacin microparticles regardless of the temperature. When the preparation time of the second fluid is 60 minutes, the differences in the degree of crystallinity/average particle diameter and the γ-type crystal ratio/average particle diameter of the obtained indomethacin microparticles is extremely large according to the temperature. When the preparation time of the second fluid is 30 minutes, although there are differences in the degree of crystallinity/average particle diameter and the γ-type crystal ratio/average particle diameter of the obtained indomethacin microparticles, there is less difference compared to when the preparation time of the second fluid is 60 minutes regardless of the preparation temperature. That is, with reference to FIG. 22 and FIG. 24, the amount of changes in the degree of crystallinity/average particle diameter and the γ-type crystal ratio/average particle diameter of the indomethacin microparticles with respect to the preparation time of the second fluid is smaller as the preparation temperature of the second fluid is higher. As an example of the control method in this case, by setting the preparation time of the second fluid after setting the preparation temperature of the second fluid high, the desired degree of crystallinity/average particle diameter and the γ-type crystal ratio/average particle diameter can be easily obtained. Further, to increase the degree of crystallinity/average particle diameter and the γ-type crystal ratio/average particle diameter with the same preparation time for the second fluid, the preparation temperature can be set low, and to decrease the degree of crystallinity/average particle diameter and the γ-type crystal ratio/average particle diameter with the same preparation time of the second fluid, the preparation temperature can be set high.

With reference to FIG. 23 and FIG. 25, by maintaining the preparation temperature of the second fluid at 33°C, which is slightly higher than room temperature, it can be understood that there are almost no differences in the degree of crystallinity/average particle diameter and the γ-type crystal ratio/average particle diameter of the obtained indomethacin microparticles regardless of the preparation time of the second fluid. When the preparation temperature of the second fluid is 5°C, the difference in the degree of crystallinity/average particle diameter and the γ-type crystal ratio/average particle diameter of the obtained indomethacin microparticles is extremely large according to the preparation time. When the preparation temperature of the second fluid is maintained at 25°C close to room temperature, although there are differences in the degree of crystallinity/average particle diameter and the γ-type crystal ratio/average particle diameter of the obtained indomethacin microparticles, there is less difference compared to when the preparation temperature of the second fluid is 5°C, depending on the length of the preparation time. That is, with reference to FIG. 23 and FIG. 25, the amount of changes in the degree of crystallinity/average particle diameter and the γ-type crystal ratio/average particle diameter of the indomethacin microparticles with respect to the prepration time of the second fluid is smaller as the preparation time of the second fluid is shorter. As an example of the control method in this case, by setting the preparation temperature of the second fluid after setting the preparation time of the second fluid short, the desired degree of crystallinity/average particle diameter and the γ-type crystal ratio/average particle diameter can be easily obtained. Further, to increase the degree of crystallinity/average particle diameter and the γ-type crystal ratio/average particle diameter with the same preparation temperature for the second fluid, the preparation time can be set longer, and to decrease the degree of crystallinity/average particle diameter and the γ-type crystal ratio/average particle diameter with the same preparation temperature of the second fluid, the preparation time can be set short.

Description of Symbols

| | |
|---|---|
| 1 | first processing surface |
| 2 | second processing surface |
| 10 | first processing member |
| 11 | first holder |
| 20 | second processing member |
| 21 | second holder |
| d1 | first introduction part |
| d2 | second introduction part |
| d20 | opening |

**Claims**

1. A method for producing microparticles, comprising:

a dissolving step of dissolving at least one type of microparticle raw material in a solvent by using an agitator having a rotating agitating blade to obtain a microparticle raw material solution; and
a separating step of separating microparticles by introducing at least one type of separating solution to separate the microparticle raw material from the microparticle raw material solution and the microparticle raw material solution in between at least two processing surfaces, which are disposed in a position facing each other, so as to be able to approach to and separate from each other, at least one of which rotates relative to the other, and mixing them in a thin film fluid formed between the at least two processing surfaces,
wherein, in the dissolving step, an agitation energy determined by an agitation time condition of the agitator, a peripheral velocity condition of the agitating blade, and a temperature condition of the microparticle raw material solution is increased or decreased by changing at least one of the conditions, and
wherein a degree of crystallinity of the microparticles obtained in the separating step is controlled by the increase and decrease of the agitation energy.

2. A method for producing microparticles, comprising:

   a dissolving step of dissolving at least one type of microparticle raw material in a solvent by using an agitator having a rotating agitating blade to obtain a microparticle raw material solution; and
   a separating step of separating microparticles by introducing at least one type of separating solution to separate the microparticle raw material from the microparticle raw material solution and the microparticle raw material solution in between at least two processing surfaces, which are disposed in a position facing each other, so as to be able to approach to and separate from each other, at least one of which rotates relative to the other, and mixing them in a thin film fluid formed between the at least two processing surfaces,
   wherein, in the dissolving step, an agitation energy determined by an agitation time condition of the agitator, a peripheral velocity condition of the agitating blade, and a temperature condition of the microparticle raw material solution is increased or decreased by changing at least one of the conditions, and
   wherein a crystal form of the microparticle obtained in the separating step is controlled by the increase and decrease of the agitation energy.

3. The method of producing microparticles according to claim 1, wherein it is controlled so that a ratio of a degree of crystallinity of the microparticles to a particle size of the microparticles increases by increasing the agitation energy in the dissolving step.

4. The method of producing microparticles according to claim 2,
   wherein the microparticle has a plurality of crystal forms, wherein a ratio of a crystalline component of a particular crystal form to a crystalline component of a plurality of crystal forms is a component ratio of the particular crystal form, and
   wherein it is controlled so that a ratio of the component ratio of the particular crystal form to the particle size of the microparticle increases by increasing the agitation energy in the dissolving step.

5. The method of producing microparticles according to any one of claims 1 to 4, wherein the microparticles are pigment microparticles.

6. The method of producing microparticles according to claim 3 or 4, wherein the separating step is for separating the microparticles by an acid pasting method, an alkaline pasting method, or a poor solvent method.

7. The method of producing microparticles according to claim 6, wherein the microparticles are pigment mircoparticles.

8. A method for producing microparticles, comprising:

   a dissolving step of dissolving at least one microparticle raw material in a solvent by using an agitator having a rotating agitating blade to obtain a microparticle raw material solution; and
   a separating step of separating microparticles by introducing at least one type of separating solution to separate the microparticle raw material from the microparticle raw material solution and the microparticle raw material solution in between at least two processing surfaces, which are disposed in a position facing each other, so as to be able to approach to and separate from each other, at least one of which rotates relative to the other, and mixing them in a thin film fluid formed between the at least two processing surfaces,
   wherein, in the dissolving step, an agitation energy determined by an agitation time condition of the agitator, a peripheral velocity condition of the agitating blade, and a temperature condition of the microparticle raw material solution is increased or decreased by changing at least one of the conditions, and
   wherein, in producing microparticles in which a particle size, a degree of crystallinity, and a crystal form are set to particular conditions,
   a first condition satisfying the particular conditions for at least one of the particle size, the degree of crystallinity, and the crystal form of the microparticle in the separating step is determined by changing one condition (the first condition) among the peripheral velocity condition, the agitation time condition, and the temperature condition in the dissolving step and fixing the other two condition (a second condition and a third condition), and
   the second and third conditions that differ from the first condition among the peripheral velocity condition, the agitation time condition and the temperature condition satisfying the particular conditions for remaining two particular conditions that are different from at least one among the particle size, the degree of crystallinity and the crystal form of the microparticle in the separating step are determined by changing at least one of the second and third conditions while maintaining the determined first condition to produce microparticles in which the particle size, the degree of crystallinity, and the crystal form satisfy the particular conditions.

Fig. 1

（A）

13

1

R

（B）

13

16

1

d20

n O

Fig. 2

Fig. 3

Fig. 4

**Fig. 5**

Fig. 6

Fig. 7

Fig. 8

Fig. 9

Fig. 10

Fig. 11

Fig. 12

Fig. 13

Fig. 14

Fig. 15

Fig. 16

Fig. 17

1/15

Fig. 19

Fig. 20

Fig. 21

Fig. 22

Fig. 23

Fig. 24

Fig. 25

## INTERNATIONAL SEARCH REPORT

| International application No. |
| --- |
| PCT/JP2015/054427 |

| A. CLASSIFICATION OF SUBJECT MATTER |
| --- |
| *B01J19/00*(2006.01)i, *C09B48/00*(2006.01)i, *C09B67/14*(2006.01)i, *C09B67/20* (2006.01)i, *A61K31/405*(2006.01)n |

According to International Patent Classification (IPC) or to both national classification and IPC

| B. FIELDS SEARCHED |
| --- |
| Minimum documentation searched (classification system followed by classification symbols)<br>B01J19/00, C09B48/00, C09B67/14, C09B67/20, A61K31/405 |

Documentation searched other than minimum documentation to the extent that such documents are included in the fields searched

Jitsuyo Shinan Koho       1922–1996    Jitsuyo Shinan Toroku Koho    1996–2015
Kokai Jitsuyo Shinan Koho   1971–2015    Toroku Jitsuyo Shinan Koho    1994–2015

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y | WO 2012/128273 A1 (M Technique Co., Ltd.), 27 September 2012 (27.09.2012), claims; paragraphs [0002], [0019], [0025] to [0027], [0029], [0064], [0081]; examples 1 to 8; table 1, 2<br>& US 2014/0001663 A1     & EP 2689839 A1<br>& CN 103429337 A        & KR 10-2014-0006935 A | 1-8 |
| Y | JP 2011-161375 A (Kansai Chemical Engineering Co., Ltd.), 25 August 2011 (25.08.2011), claims; paragraphs [0002], [0012], [0024], [0027] to [0029]<br>(Family: none) | 1-8 |

| ☒   Further documents are listed in the continuation of Box C. | ☐   See patent family annex. |
| --- | --- |

| *   Special categories of cited documents: | "T"   later document published after the international filing date or priority date and not in conflict with the application but cited to understand the principle or theory underlying the invention |
| --- | --- |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search<br>    25 March 2015 (25.03.15) | Date of mailing of the international search report<br>    07 April 2015 (07.04.15) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (July 2009)

**INTERNATIONAL SEARCH REPORT**

International application No.

PCT/JP2015/054427

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2000-72436 A  (Mitsubishi Chemical Corp.), 07 March 2000 (07.03.2000), claims; paragraphs [0004], [0020], [0022], [0023] (Family: none) | 1-8 |
| A | JP 2012-157858 A  (M Technique Co., Ltd.), 23 August 2012 (23.08.2012), claims (Family: none) | 1-8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002097281 A
- JP 2006193652 A
- WO 2009008393 A
- WO 2012128273 A
- JP 4691698 B
- JP 4419157 B

**Non-patent literature cited in the description**

- **MARTINDALE.** The Extra Pharmacopoeia. The Pharmaceutical Press, 1989